(19) Europäisches Patentamt / European Patent Office / Office européen des brevets

(11) **EP 2 022 453 B1**

(12) **EUROPEAN PATENT SPECIFICATION**

(45) Date of publication and mention
of the grant of the patent:
**17.02.2016 Bulletin 2016/07**

(21) Application number: **07742953.8**

(22) Date of filing: **08.05.2007**

(51) Int Cl.:
*A61F 13/49* (2006.01)       *A61F 13/15* (2006.01)
*A61F 13/496* (2006.01)      *A61F 13/514* (2006.01)

(86) International application number:
**PCT/JP2007/059518**

(87) International publication number:
**WO 2007/138821 (06.12.2007 Gazette 2007/49)**

(54) **DISPOSABLE PULL-ON DIAPER**

EINWEG-HÖSCHENWINDEL

COUCHE-CULOTTE À ENFILER JETABLE

(84) Designated Contracting States:
**DE FR GB SE**

(30) Priority: **25.05.2006  JP 2006144753**
           **08.08.2006  JP 2006215402**

(43) Date of publication of application:
**11.02.2009 Bulletin 2009/07**

(73) Proprietor: **Kao Corporation
Chuo-ku,
Tokyo 103-8210 (JP)**

(72) Inventors:
• **SASAKI, Jun
Haga-gun
Tochigi 321-3497 (JP)**
• **ONDA, Aiko
Haga-gun
Tochigi 321-3497 (JP)**

• **YANASHIMA, Takuo
Haga-gun
Tochigi 321-3497 (JP)**

(74) Representative: **Vossius & Partner
Patentanwälte Rechtsanwälte mbB
Siebertstrasse 3
81675 München (DE)**

(56) References cited:
**EP-A2- 0 873 738       EP-A2- 1 159 942
WO-A1-02/34185         WO-A1-2005/122984
JP-A- 05 076 565       JP-A- 09 192 169
JP-A- 10 016 115       JP-A- 11 216 160
JP-A- 11 276 523       JP-A- 2002 209 942
US-A1- 2002 193 774**

**Description**

Technical Field

The present invention relates to a disposable pull-on diaper and a method for producing the same.

Background Art

Known disposable diapers include the type having an absorbent body including an absorbent core and a stretch sheet as an outer cover joined to the garment-facing side of the absorbent body and providing a better fit against the body of a wearer (see, e.g., JP 3-205053A and JP-U 4-32718A). Not using linear elastic materials, such as rubber threads, for imparting stretchability to the outer cover, the disposable diapers of this type are superior in appearance and softness before and during use and also eliminate the problem of leaving an elastic mark on the wearer's skin.

This type of disposable diapers, however, often involve the problem that the diaper is not easy to put on a wearer or provides a poor fit during wear because the stretchability of the stretch outer cover is hindered by the absorbent body having substantially no stretchability. Then it has been proposed to reduce the joint area between the absorbent body and the outer cover as disclosed, e.g., in JP 62-162001A.

The disposable diaper of JP 62-162001A succeeds to some extent in alleviating the above problem by reducing the joint area between the absorbent body and the outer cover. However, because the joint area of the outer cover with the absorbent body has stretchability, there arises the problem that the outer cover tends to separate from the absorbent body or break when extended in the lateral direction while worn. In addition to this problem, further improvement on the softness of the skin facing side of an outer cover has been demanded.

US 2002/0193774 A1 describes a disposable diaper having a backsheet comprising an elastic inner layer and an inelastic outer layer. The elastic inner layer, in a transversely stretched state, is joined to the inelastic outer layer at a plurality of intermittently disposed spots.

The present invention relates to a disposable pull-on diaper having an absorbent body containing an absorbent core and an outer cover joined to the garment facing side of the absorbent body, the outer cover being free from the above described inconveniences, such as separation from the absorbent body when extended in the lateral direction, and having good softness on its skin facing side. The invention also provides a method of producing such a diaper. The present invention is defined by the features of the claims.

The present invention provides a disposable pull-on diaper including an absorbent body containing an absorbent core and an outer cover joined to the garment facing side of the absorbent body. The outer cover includes a front portion and a rear portion and has both side edge portions of the front portion and both side edge portions of the rear portion jointed together to form a pair of side seals, a waist opening, and a pair of leg openings. The outer cover is a laminate composed of an outer sheet formed of a stretch sheet and an inner sheet formed of a non-stretch sheet. The outer sheet and the inner sheet are not joined to each other in the entire or almost the entire area of the outer cover except the side seals, a peripheral portion around the waist opening, and a peripheral portion around each of the leg openings. The absorbent body and the inner sheet of the outer cover are joined together via a joint region.

The present invention also provides a method for producing the above described disposable pull-on diaper in which the substantial width of the inner sheet is larger than the width of the outer sheet with the side seals taken apart, the pair of side seals in an unfolded state, and the outer sheet in a relaxed state. The method includes the steps of joining the outer sheet in a laterally extended state to the inner sheet in a laterally flattened state, joining the absorbent body to the inner sheet while maintaining the outer sheet in the extended state, and releasing the outer sheet from the extending force to have the outer sheet retract by its own retractability.

Brief Description of the Drawing

Fig. 1 is a perspective showing a first embodiment of the disposable pull-on diaper according to the present invention.
Fig. 2 is a plan of the disposable diaper of Fig. 1 in its flat-out state.
Fig. 3 is a developed perspective of the disposable diaper of Fig. 1.
Fig. 4 is a cross-section taken along line IV-IV of Fig. 2.
Fig. 5(a) is a plan of the absorbent core used in the disposable diaper shown in Fig. 1.
Fig. 5(b) is a cross-section taken along line B-B in Fig. 5(a).
Fig. 5(c) is a cross-section taken along line C-C in Fig. 5(a).
Fig. 6 is a perspective view illustrating a first embodiment of the method of producing a disposable pull-on diaper according to the present invention.

Fig. 7 is a perspective of a second embodiment of the disposable pull-on diaper according to the present invention.

Fig. 8 is a plan of the disposable diaper of Fig. 7 in its flat-out state.

Fig. 9 is a developed perspective of the disposable diaper of Fig. 7.

Fig. 10 is a cross-section taken along line IV-IV of Fig. 8.

Fig. 11 is a perspective view illustrating an embodiment of the method of producing a disposable pull-on diaper shown in Fig. 7.

Fig. 12(a) is a plan schematically showing a pattern of a joint region between outer and inner sheets.

Fig. 12(b) is a plan schematically showing another pattern of a joint region between outer and inner sheets.

Fig. 13(a) is a plan schematically showing a spiral pattern of a joint region between outer and inner sheets.

Fig. 13(b) is a schematic plan used to describe the joint area of the spiral joint region.

Fig. 14(a) is a schematic bottom view of an absorbent body, illustrating a pattern of a joint region between the absorbent body and an outer cover.

Fig. 14(b) is a schematic bottom view of an absorbent body, illustrating another pattern of a joint region between the absorbent body and an outer cover.

Fig. 15(a) is a schematic bottom view of an absorbent body, illustrating a spiral joint region between the absorbent body and an outer cover.

Fig. 15(b) is a schematic bottom view used to describe the joint area of the spiral joint region.

Detailed Description of the Invention

The disposable pull-on diaper of the invention will be described based on a preferred first embodiment with reference to the accompanying drawing.

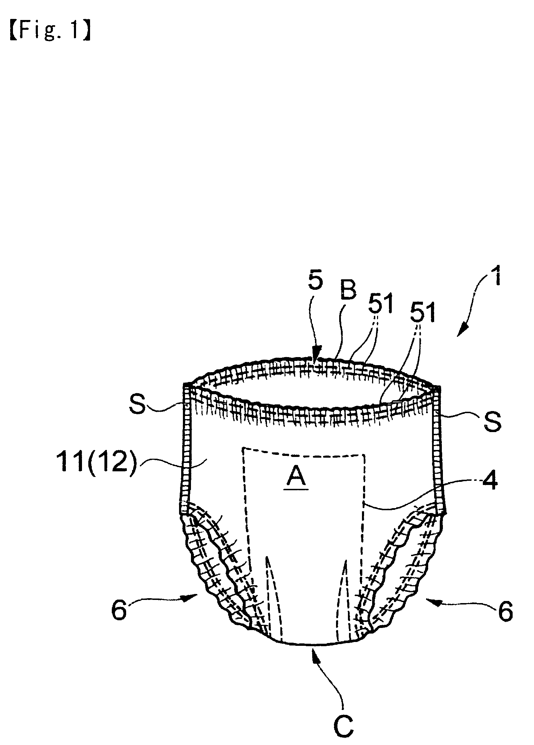

As illustrated in Figs. 1 through 4, the disposable diaper 1 of the first embodiment includes a liquid permeable topsheet 2, a liquid impermeable or water repellent backsheet 3, a substantially oblong absorbent body 10 interposed between the topsheet 2 and the backsheet 3, and an outer cover 11 joined to the side of the backsheet 3 (i.e., the garment facing side) of the absorbent body 10. The absorbent body 10 contains a liquid retentive absorbent core 4.

The outer cover 11 has a longitudinally middle portion thereof narrowed to have the shape of a sandglass and defines the outline of the diaper. The outer cover 11 is sectioned into a front portion A designed to be applied to the stomach side of a wearer, a rear portion B designed to be applied to the rear side of a wearer, and a crotch portion C located between the front portion A and the rear portion B. The front portion A and the rear portion B correspond to the longitudinal end portions of the outer cover 11, and the crotch portion C corresponds to the longitudinally middle portion of the outer cover 11.

The front portion A and the rear portion B of the outer cover 11 are connected to each other in both side edge portions A1 and A2 of the former and both side edge portions B1 and B2 of the latter, forming a pair of side seals S. So connected, the outer cover 11 forms the shape of a pair of pants having a waist opening 5 and a pair of leg openings 6. The connection of the sides is achieved by, for example, heat sealing, high frequency sealing, or ultrasonic sealing.

The topsheet 2, the backsheet 3, and the absorbent core 4 are each rectangular and united into an oblong absorbent body 10. The topsheet 2, the backsheet 3, and the absorbent core 4 are of conventional types commonly used in the art. For example, the absorbent core 4 may be an aggregate made up of superabsorbent polymer particles and a fibrous material and wrapped in tissue paper (not shown).

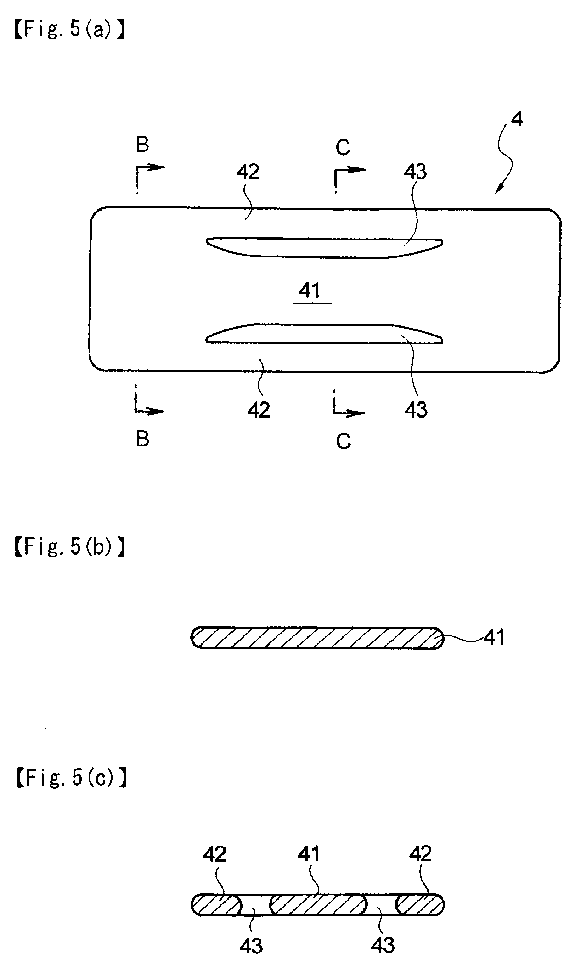

As illustrated in Figs. 5(a) through 5(c), the absorbent core 4 used in the first embodiment includes a sandglass-shaped central absorbent member 41 and a pair of side absorbent members 42 on both sides of the central absorbent member 41 in a symmetrical configuration. The central absorbent member 41 is separate from the side absorbent members 42 in at least the crotch portion. One longitudinal end portion and the other longitudinal end portion of each side absorbent member 42 are continuous with one longitudinal end portion (front portion) and the other longitudinal end portion (rear portion), respectively, of the central absorbent member 41. Accordingly, a closed gap 43 is formed between the central absorbent member 41 and each of side absorbent members 42.

One longitudinal end portion, the other longitudinal end portion, and a longitudinally middle portion approximately correspond to one-third of the length of the absorbent core 4.

The side edges of the absorbent core 4 rise easily because of the gaps 43. When the absorbent core 4 is pressed laterally inwardly, the overall width of the absorbent core 4 reduces because of the gaps 43, which is less likely to impede lateral contraction of the outer cover 11.

When the diaper 1 is put on a wearer with the waist opening 5 widened, the rise of both side edges of the absorbent core 4 reduces the width of the absorbent body 10 viewed from the skin facing side of the diaper 1, which helps a diaperer to insert the wearer's leg into the leg opening.

The plan view shape of the absorbent core 4 is not limited to the one shown in Figs. 5(a) to 5(c). While not shown, the side absorbent members 42 may be continuous with only one longitudinal end portion of the central absorbent member 41, or side absorbent members 42 may be separated from the central absorbent member 41, or the absorbent core 4 may have no gaps 43.

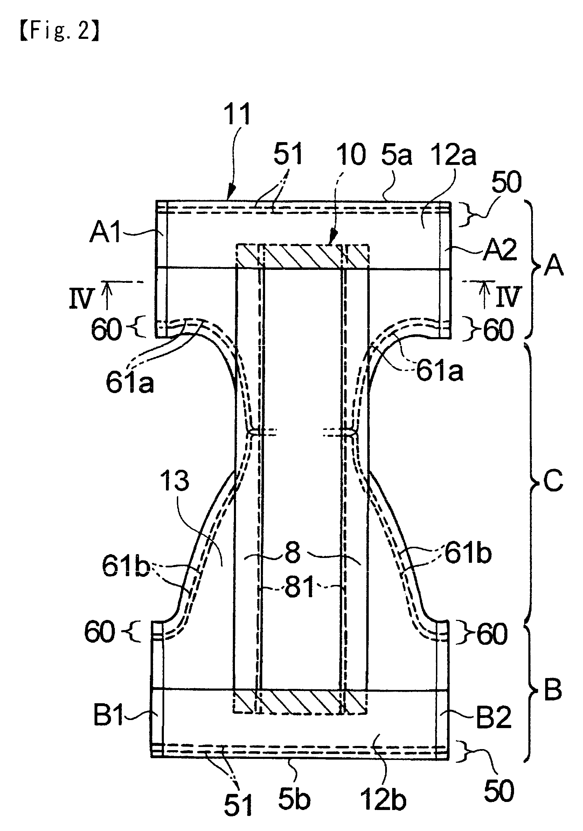

A side cuff 8 formed of a liquid resistant or water repellent and air permeable material is provided along both sides of the absorbent body 10 as illustrated in Figs. 2 to 4. A cuff elastic member 81 is disposed in its stretched state along near the free edge of each side cuff 8. When the assembled disposable diaper 1 of Fig. 1 is put on a wearer, the cuff elastic members 81 contract to raise the side cuffs 8, thereby to impede the lateral flow of body fluids.

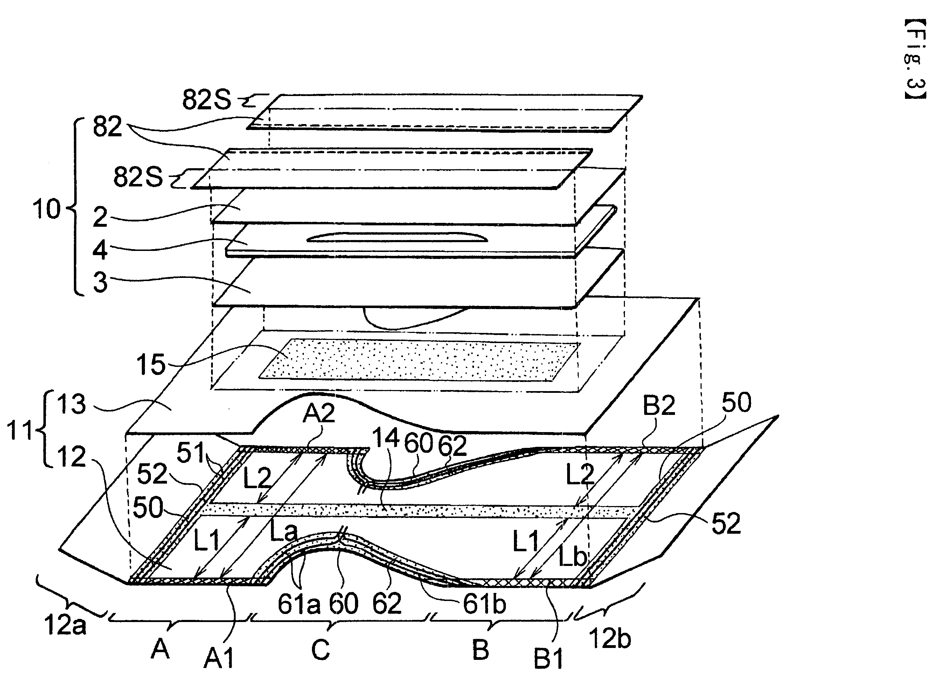

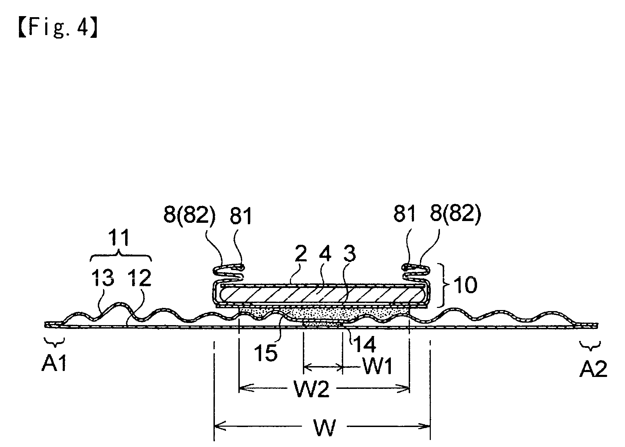

The side cuff 8 is formed of a cuff-forming sheet 82. As illustrated in Figs. 3 and 4, the cuff-forming sheet 82 extends laterally outward from the absorbent body 10 to a prescribed width to provide an extension 82S, which, in an assembled state, wraps around beneath the absorbent core 4 and is secured between the absorbent core 4 and the skin facing side of the backsheet 3.

The outer cover 11 has a laminate structure composed of an outer sheet 12 formed of a stretch sheet and an inner sheet 13 formed of a non-stretch sheet. The outer sheet 12 provides the exterior surface of the diaper, and the inner sheet 13 is disposed on the inner side of the outer sheet 12. It is only necessary for the stretch sheet forming the outer sheet 12 to be stretchable in at least the lateral direction (the horizontal direction in Fig.2) of the diaper in an unfolded state. The non-stretch sheet forming the inner sheet 13 has no stretchability in at least the lateral direction of the diaper with the diaper in an unfolded state.

The term "stretchability" or "stretchable" as used herein with respect to a stretch sheet means the ability of an extensible sheet having a maximum elongation of at least 100% in at least one direction (i.e., extensible to at least twice the original length) to retract to a recovery of at least 70% when extended in that direction to an elongation of 100%. In the case of a stretch sheet having a maximum elongation of 100% or more in two or more directions, it is only necessary for the sheet to have a recovery from extension of at least 70% in any one of the directions.

The outer sheet 12 is more extensible in the diaper lateral direction than in the longitudinal direction (the vertical direction in Fig. 2). Specifically, the outer sheet 12 is extensible largely (having a maximum elongation of 100% or more) in the lateral direction and retractable (having a recovery of 70% or more) when released, whilst it is extensible only slightly (e.g., having a maximum elongation of 50% or less) in the diaper longitudinal direction.

The recovery from extension is measured as follows.

Method of measuring recovery from extension:

A 25 mm wide and 50 mm long cut sample of a stretch sheet is set on a tensile tester (Tensilon RTC-1150A, from Orientec Co., Ltd.) at an initial jaw separation of KO (mm) and extended 100% to a length K2 (K2=K0 x 2) at a pulling rate of 300 mm/min. The sample is then retracted at the same rate. When the stress returned to zero, the length K1 of the sample (the length after recovery from extension) is measured. The recovery from 100% extension is calculated from the following formula:

$$\text{Recovery from 100\% extension (\%)} = [(K2 - K1)/(K2 - K0)] \times 100$$

The outer sheet 12 is formed of various sheets with stretchability. In terms of good breathability, for instance, the outer sheet 12 is preferably formed of nonwoven fabric made of thermoplastic fibers. In terms of good hand, the outer sheet 12 is preferably formed of a laminate sheet having air-through nonwoven fabric on at least the garment facing side thereof. A stretch sheet having elastically stretchable fibers (elastomeric fibers) on the garment facing side thereof feels slimy to the touch and is unfavorable in terms of hand.

More detailed preferred examples of the stretch sheet that forms the outer sheet 12 include (1) a sheet having an elastic fiber layer and an extensible fiber layer united to one or both sides of the elastic fiber layer, (2) a sheet having an elastic net sheet and an extensible fiber layer united to one or both sides of the elastic net sheet, and (3) a sheet having an elastic film and an extensible fiber layer united to one or both sides of the elastic film. Uniting the elastic fiber layer and the extensible fiber layer is carried out by, for example, entangling the fibers of the two fiber layers superposed on each other by hydroentanglement or through-air bonding or bonding the two fiber layers by heat embossing, applying an adhesive, or ultrasonication.

The sheet (1) is exemplified by (a) a stretch nonwoven fabric having an elastic fiber layer and a substantially inelastic, inelastic fiber layer on at least one side of the elastic fiber layer. The elastic fiber layer and the inelastic fiber layer are joined together all over by fusion bonding at fiber intersections while the fibers constituting the elastic fiber layer remain in a fibrous form. The inelastic fiber layer has part of its fibers enter the elastic fiber layer and/or the elastic fiber layer has part of its fibers enter the inelastic fiber layer.

Exemplary and preferred of the sheets (1), (2), and (3) include (b) a stretch sheet having an elastically stretchable elastic layer and a substantially inelastic, inelastic fiber layers, which is obtained by superposing the two layers on each other, partially joining them, and stretching the resulting laminate sheet.

The stretch nonwoven fabric (a) is described in more detail. The fibers constituting the elastic fiber layer and the fibers constituting the inelastic fiber layer are fusion bonded to each other at their intersections on and near the interfaces between the elastic fiber layer and the inelastic fiber layer. Thus, the fiber layers are evenly joined together substantially all over their interfaces. Being joined all over, the fiber layers are prevented from separating from each other and forming a gap therebetween. The stretch nonwoven fabric has a multilayer structure and yet exhibits integrity like monolithic nonwoven fabric.

By the expression "the constituent fibers of the elastic fiber layer remain in a fibrous form" is meant that most of the fibers constituting the elastic fiber layer are not in a cohesive film-like state or a cohesive film-like/fibrous mixed state even after application of heat, pressure and so forth. The elastic fiber layer has its fibers fusion bonded at their intersections throughout its thickness. Likewise, the inelastic fiber layer has its fibers fusion bonded at their intersections throughout their thickness.

In the case where the inelastic fiber layer is provided on both sides of the elastic fiber layer, at least one of the inelastic fiber layers has part of its constituent fibers enter the elastic fiber layer and/or the elastic fiber layer has part of its constituent fibers enter at least one of the inelastic fiber layers. In order to have the fibers of the inelastic fiber layer enter the elastic fiber layer and/or to have the fibers of the elastic fiber layer enter the inelastic fiber layer, it is desirable that at least one of the inelastic fibers and the elastic fibers be in the form of a web, i.e., a loose aggregate of fibers having no fusion bonds before the step of fusion bonding the fibers of the inelastic fiber layer and the fibers of the elastic fiber layer. To help fibers of a layer to enter another layer, it is desirable that the web be made up of staple fibers for higher freedom of movement than continuous fibers.

A through-air process is preferred for having the fibers of the inelastic fiber layer enter the elastic fiber layer and/or having the fibers of the elastic fiber layer enter the inelastic fiber layer. A through-air process easily achieves having fibers of a layer enter another layer facing thereto and having a layer receive fibers from another layer facing thereto. A through-air process easily achieves having the fibers of the inelastic fiber layer enter the elastic fiber layer while retaining the bulk of the inelastic fiber layer. In the cases where the fibers of the inelastic fiber layer are entangled with the fibers of the elastic fiber layer, it is preferred that the entanglement be achieved only by a through-air process. Fiber entanglement by a through-air process is preferably accomplished by properly adjusting the air blowing pressure, air velocity, basis weight and thickness of the fiber layers, the running speeds of the fiber layers, and so on. The through-air treatment is preferably carried out under specific conditions.

When the inelastic fiber web and an elastic fiber web are treated with hot air (particularly in a through-air system), part of the fibers of the inelastic fiber web enter the elastic fiber web and, at the same time, the fibers of the inelastic fiber web and/or the fiber of the inelastic fiber web and the fibers of the elastic fiber web are fusion bonded at their intersections. In this situation, care should be taken that the fibers of the elastic fiber web do not assume a cohesive film-like state or a cohesive film-like/fibrous mixed state as a result of the hot air treatment.

The elastic fiber layer has the capability of extending under tension and retracting or contracting when released from the tension. When the elastic fiber layer is 100% elongated in at least one direction parallel to its plane and then retracted, the residual strain is preferably 20% or less, more preferably 10% or less. It is desirable that the elastic fiber layer has the recited residual strain in at least the diaper lateral direction, particularly preferably in both the diaper lateral and longitudinal directions.

The elastic fiber layer is an aggregate of elastic fibers. Methods of forming elastic fibers include a melt-blowing method in which a molten resin is extruded through orifices and the extruded molten resin stream is drawn by hot air into fine fibers, a spun bonding method in which a half-molten resin is drawn by cool air or by mechanical drawing, and a blow spinning method, which is a combination of a melt blowing method and a spun bonding method and may be said to be a modified melt blowing method.

The elastic fiber layer may have the form of a web or nonwoven fabric made of elastic fibers by, for example, blow spinning, spun bonding or melt blowing. The elastic fiber layer is particularly preferably a web obtained by blow spinning.

The fibers that can be used to constitute the elastic fiber layer include those made from thermoplastic elastomers or rubber. Thermoplastic elastomers are melt-spinnable using an extruder in the same manner as ordinary thermoplastic resins, and the fibers thus obtained are easy to fusion bond. Therefore, fibers of thermoplastic elastomers are particularly suited for making the stretch nonwoven fabric that has air-through nonwoven as a basic structure.

Examples of the thermoplastic elastomers include styrene elastomers such as SBS (styrene-butadiene-styrene block copolymer), SIS (styrene-isoprene-styrene block copolymer), SEBS (styrene-ethylene-butylene-styrene block copolymer), and SEPS (styrene-ethylene-propylene-styrene copolymer), olefin elastomers, polyester elastomers, and polyurethane elastomers. These elastomers may be used either individually or in combination of two or more thereof.

The inelastic fiber layer is extensible but substantially inelastic. The term "extensible" as used herein is intended to include not only a fiber layer whose constituent fibers *per se* are extensible but also a fiber layer whose constituent fibers are not *per se* extensible but which shows extensibility as a result of debonding of constituent fibers that have been fusion bonded at their intersections, change of a three-dimensional structure formed of a plurality of constituent fibers fusion-bonded to one another, or breaks of the constituent fibers.

The fibers that can be used to constitute the inelastic fiber layer include fibers of polyethylene (PE), polypropylene (PP), polyesters (PET and PBT), and polyamides. The fibers constituting the inelastic fiber layer may be staple fibers or continuous fibers and hydrophilic or water repellent. Sheath-core or side-by-side conjugate fibers, split fibers, modified cross-section fibers, crimped fibers, and heat shrunken fibers are also useful. These fibers may be used either individually or in combination of two or more thereof. The inelastic fiber layer may be a web or nonwoven fabric of continuous filaments or staple fibers.

The stretch sheet (b) will be described in more detail. The stretch sheet (b) is obtained by providing a laminate sheet having an elastically stretchable elastic layer and a substantially inelastic, inelastic fiber layer bonded to one or both sides of the elastic layer in parts in a regular pattern and stretching the laminate sheet.

The laminate sheet is prepared, for example, as follows. A fiber web (first inelastic fiber layer) is continuously fed from a carding machine. Elastic fibers are fed on the fiber web to form an elastic layer. A fiber web (second inelastic fiber layer) fed from another carding machine is continuously fed on the elastic layer. The resulting stack of three layers is subjected to a hot air treatment in a through-air system drier. The hot air treated laminate sheet is heat embossed through an embossing unit including an embossing roller having embossing projections regularly arranged on its peripheral surface and an anvil roller facing to the embossing roller. In the process described above, the hot air treatment in the drier is for causing the elastic fibers and the inelastic fibers to be fusion bonded or to mutually enter the adjoining fiber layer. The hot air treatment may be omitted.

The elastic layer preferably contains elastic fibers made from an elastic material. Elastic materials include thermoplastic elastomers, rubber, and ethylene-propylene copolymers. Thermoplastic elastomers are preferred of them; for they are relatively easily formed into elastic fibers. Examples of the thermoplastic elastomers include polyurethane elastomers, styrene elastomers (e.g., SBS, SIS, SEBS, and SEPS), olefin elastomers (e.g., ethylene, propylene or butene copolymers), vinyl chloride elastomers, and polyester elastomers. These elastomers may be used either individually or in combination of two or more thereof.

The proportion of the elastic fibers made from an elastic material in the elastic layer is preferably 50% to 100% by weight, more preferably 75% to 100% by weight. The elastic layer may be a film or a net instead of the fiber layer. The film or net can be made from the above recited elastic materials.

The non-stretch sheet forming the inner sheet 13 will then be described. As used herein the term "non-stretch sheet" refers to a sheet having a recovery (%) from extension of 20% or less as measured by the above descried method of measuring recovery from extension or a sheet that breaks before being 100% extended. The non-stretch sheet may be extensible or inextensible but preferably has a maximum elongation of 90% or less.

Exemplary and preferred of the non-stretch sheets are nonwoven fabric, laminates of nonwoven fabric and resin film, and porous film. To secure good breathability and hand, the non-stretch sheet is preferably nonwoven fabric made of thermoplastic fibers. To secure prevention of leakage of body wastes, the non-stretch sheet is preferably water repellent nonwoven fabric.

The outer sheet 12 generally has low sheet strength so that the outer cover 11 can tear while worn or be broken through with the finger of a diaperer when in putting the diaper on a wearer. To avoid this, it is more desirable for the inner sheet 13 to have higher strength. Specifically, the inner sheet 13 preferably has a breaking strength of 5 N/25 mm or higher in both the diaper lateral and longitudinal directions.

The breaking strength is measured by the following method.

Method of measuring breaking strength:

A sample measuring 25 mm in width and 50 mm in length is set on a tensile tester at an initial jaw separation of 30 mm and pulled at a rate of 300 mm/min. The maximum load applied is taken as a breaking strength of the sample in the pulling direction. The measurement is taken in quintuplicate for each of the lateral and the longitudinal directions of the diaper to obtain an average.

The inner sheet 13 is preferably formed of nonwoven fabrics produced by a spun bonding method, a heat roller method, and the like in view of breaking strength, breathability, and prevention of strike-through of an adhesive.

In the disposable diaper 1 of the first embodiment, as illustrated in Figs. 3 and 4, the outer sheet 12 and the inner sheet 13 are joined to each other in the side edge portions A1, A2, B1, and B2 by heat sealing, high frequency sealing or ultrasonic sealing and in the peripheral portion 50 along the waist opening 5 and the peripheral portion 60 along each leg opening 6 by means of an adhesive 52 and 62 (e.g., a hot melt adhesive), respectively. The outer sheet 12 and the inner sheet 13 are not joined in the most of the area of the outer cover except these portions in the front portion A and the rear portion B.

Specifically, the outer sheet 12 and the inner sheet 13 are joined not only in the side edge portions A1, A2, B1, and B2, the peripheral portion 50 along the waist opening 5, and the pair of peripheral portions 60 along the leg openings 6 but also in the laterally middle portion in the front portion A and the rear portion B. The outer sheet 12 and the inner sheet 13 are not joined in other than these portions.

The outer sheet 12 and the inner sheet 13 being not joined over a broad area in the front portion A and the rear portion B, the area in which the outer cover 11 becomes stiff due to an adhesive applied can be minimized, and the diaper feels soft and pleasant to the touch on its exterior surface and the exposed part (the part not covered by the absorbent body 10) of the inner surface of the outer cover 11.

When the outer sheet 12 and the inner sheet 13 are formed of breathable sheets, there is provided a diaper having good breathability and thereby capable of controlling the inside humidity. The outer sheet 12 and the inner sheet 13 being joined in the peripheral portions 50 and 60 along the waist opening 5 and the leg openings 6, the stretchable outer cover 11 provides moderate fit in the portion below the waist opening 5.

The peripheral portion 50 along the waist opening 5 in each of the front portion A and the rear portion B has a plurality of waist elastic members 51 disposed along the edge of the waist opening 5. The waist elastic members 51 are fixed in their stretched state between the outer sheet 12 and the inner sheet 13 via the adhesive 52.

The peripheral portion 60 along each of the leg openings 6 straddling the front portion A and the rear portion B has leg elastic members 61 a and 61b disposed along the edge of the leg opening 6. The leg elastic members 61a and 61b are secured between the outer sheet 12 and the inner sheet 13 in their stretched state by the adhesive 62.

The waist elastic members 51 and the leg elastic members 61a and 61b are preferably rubber threads or tapes made from stretchable materials, such as natural rubber, polyurethane resins, foamed urethane resins, and hot-melt extensible materials.

With the elastic members 51, 61 a, and 61b of thread or tape form being fixed in the peripheral portions 50 and 60 along the waist opening 5 and the leg openings 6 as sandwiched in between the outer sheet 12 and the inner sheet 13, these peripheral portions may be designed to have improved fit without being restricted by the stretch characteristics of the outer cover 11.

With the elastic members 51, 61a, and 61b of thread or tape form being fixed as sandwiched in between the outer sheet 12 and the inner sheet 13, there is less likelihood of causing a discomfort to a wearer or spoiling the diaper appearance than when the elastic members are fixed to an outer cover formed of a single sheet.

In the disposable diaper 1 of the first embodiment, the width of the peripheral portion 50 along the waist opening 5 where the outer sheet 12 and the inner sheet 13 are joined is preferably within 70 mm, more preferably within 60mm, from the edges 5a and 5b of the waist opening in the front portion A and the rear portion B. The width of the peripheral portion 60 along the leg opening 6 where the outer sheet 12 and the inner sheet 13 are joined is preferably within 50 mm, more preferably within 30 mm, from the edge of the leg opening 6 in each of the front portion A, the crotch portion C, and the rear portion B.

The regions in the front portion A and the rear portion B where the outer sheet 12 and the inner sheet 13 are not joined together preferably have a total length in the diaper lateral direction, L1+L2, of 60% or more, more preferably 70% or more, even more preferably 75% or more, of the length La (or Lb) between the laterally opposing side edge portions A1 and A2 (or B1 and B2).

The region in each of the front portion A and the rear portion B where the outer sheet 12 and the inner sheet 13 are not joined preferably has a total area of 60% to 100%, more preferably 70% to 100%, of the area of the outer sheet 12 in the front portion A or the rear portion B. When the area of the region where the outer sheet 12 and the inner sheet 13 are not joined falls within the above recited range, the area is referred to as "the entire or almost the entire area".

The dimensions, ratios of dimensions, and the like as referred to in the first embodiment are those measured or calculated on a diaper in a flat-out state with all the elastic members along the waist opening and the leg openings being in a relaxed state with no contractibility (with no outer force, like tension, applied thereto) as illustrated in Fig. 2.

In the crotch portion C of the diaper 1 of the first embodiment, the outer sheet 12 and the inner sheet 13 are joined to each other in the peripheral portions 60 along the leg openings 6 via the adhesive 62 and in the laterally middle portion via a joint region 14. These two sheets are not joined in other than these portions in the crotch portion C.

As illustrated in Fig. 2, the leg elastic members 61 for elasticizing the peripheral portion 60 of the leg opening in the crotch portion extend laterally inwardly from the peripheral portion 60 of the leg opening. This inwardly extending part having the leg elastic members 61 disposed to elasticize in the peripheral direction of the leg opening is also included within the term "peripheral portion 60" of the leg opening.

In the disposable diaper 1 of the first embodiment, since the outer sheet 12 and the inner sheet 13 are joined along the laterally middle portion in the front portion A, rear portion B, and crotch portion C, the diaper has improved appearance both before and during use. In the case where a tape for disposal (not shown) is provided on the exterior surface of the outer cover 11, the tape is firmly secured to the laterally middle portion. The tape for disposal is means to hold a used diaper in a rolled condition. Various conventional tapes for disposal can be used.

The outer sheet 12 and the inner sheet 13 being joined along the laterally middle portion in the front portion A, rear portion B, and crotch portion C, a pattern, a letter, or any other design, if printed on the backsheet 3, is easily seen through the outer cover.

As illustrated in Figs. 2 and 3, the outer sheet 12 providing the exterior side of the outer cover 11 extends longitudinally outwardly from the positions where the waist elastic members 51 are held between the outer sheet 12 and the inner sheet 13 to form extensions 12a and 12b extending from the inner sheet 13. The extensions 12a and 12b are folded back over the side of the absorbent body 10.

The folded extensions 12a and 12b of the outer sheet 12 cover the longitudinal end portions of the absorbent body 10. An adhesive (not shown) is applied between the overlapping part of the folded extensions 12a and 12b and the longitudinal end portions of the absorbent body 10 over the whole width of the absorbent body 10 so that the longitudinal end portions of the absorbent body 10 is secured to the outer cover 11.

The folded extensions 12a and 12b prevent the front and rear ends of the absorbent body 10 from coming into direct contact with the wearer's body and prevent leakage of the absorbent polymer of the absorbent core 4 from the front and rear ends of the absorbent body 10.

As illustrated in Figs. 3 and 4, the part of the absorbent body 10 other than the longitudinal end portions thereof is joined to the inner sheet 13 of the outer cover 11 in a joint region 15 provided on the laterally middle portion thereof. In view of preventing the absorbent body 10 from separating from the outer cover 11, the total area T2 of the joint region 15 to the area T1 of the outer cover-facing surface of the absorbent body 10, T2/T1, is preferably at least 60%, more preferably 65% or more, with the outer cover 11 being in a relaxed stated.

The joint region 14 between the outer sheet 12 and the inner sheet 13 in the laterally middle portion of the outer cover 11 preferably has a width W1 of 0 to 40%, more preferably 0 to 30%, of the width W of the absorbent body 10 in each of the front portion A and the rear portion B. If the width W1 of the joint region 14 exceeds 40% of the width W of the absorbent body 10, the stretchability of the outer cover is impeded.

The joint region 15 is an elongated rectangular region extending in the longitudinal direction in the laterally middle portion of the absorbent body 10. The width W2 of the joint region 15 is preferably at least 70%, more preferably 75% or more, of the width W of the garment facing side of the absorbent body 10 in each of the front portion A and the rear portion B. With the width W2 of the joint region 15 being 70% or more of the width W of the absorbent body 10, the absorbent body 10 is less likely to separate or break during wear.

In the first embodiment, when the outer cover 11 is in an unfolded, flat-out state with the side seals S taken apart, the substantial width of the inner sheet 13 is larger than the width of the outer sheet 12 in a relaxed state. When the outer cover 11 is in an unfolded state, the outer sheet 12 is in a contracted state due to its retractability, while the inner sheet 13, being formed of a non-stretch sheet, does not virtually reduce in width. As a result, the inner sheet 13 is loose in the lateral direction relative to the outer sheet 12. By the expression "substantial width of the inner sheet 13" as used herein is meant the width of the inner sheet 13 imagiriarily separated from the outer sheet 12 and flattened out.

The substantial width of the inner sheet 13 is preferably 1.3 to 4.0 times, more preferably 1.5 to 3.0 times, the width of the outer sheet 12 in its relaxed state. The outer cover 11 having such a structure is prepared by, for example, the method described *infra*.

In the so designed disposable pull-on diaper of the first embodiment, the substantial width of the inner sheet 13 is larger than the width of the outer sheet 12 with the side seals S taken apart, the outer cover 11 unfolded, and the outer sheet 12 in the relaxed state. By virtue of this configuration as well as the fact that the outer sheet 12 of the outer cover 11 is formed of a stretch sheet, the diaper has a neat appearance and a good fit while worn.

Since the inner sheet 13 of the outer cover 11 is formed of a non-stretch sheet, even when the outer cover 11 is extended in the lateral direction on use, the inner sheet 13 is slackened, and there is less likelihood that the absorbent body 10 separates from the outer cover 11 or the outer cover 11 breaks. Furthermore, the skin contacting surface of the outer cover 11 has good feel and hand with excellent softness.

The outer sheet 12 formed of a stretch sheet and the inner sheet 13 formed of a non-stretch sheet not being joined to each other in the most part of the outer cover 11, the outer cover 11 exhibits good stretchability with no influences from the non-stretchable absorbent body 10. When the outer sheet 12 of the outer cover 11 is released from the extended state, the absorbent body 10 continues having a good appearance and high absorptivity without being bunched up. Therefore, the lateral stretchability of the outer cover 11 is not impeded even if the absorbent body 10 is designed to have a wide the width W2 of the joint region 15.

The method for producing the disposable pull-on diaper of the present invention will then be described based on a preferred first embodiment thereof. The method of the first embodiment is for producing the disposable pull-on diaper 1 of the first embodiment in a continuous manner.

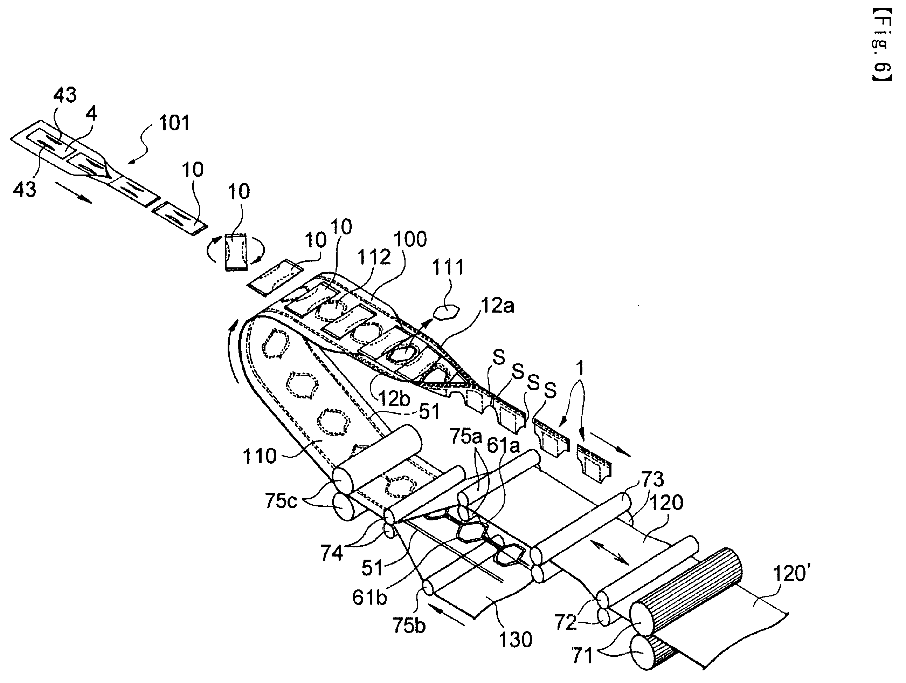

As illustrated in Fig. 6, the method of the first embodiment includes joining an outer sheet 12 in its laterally extended state to an inner sheet 13 in its flattened state, joining an absorbent body 10 to the inner sheet 13 via a joint region (not shown) while maintaining the outer sheet 12 in the extended state, and releasing the outer sheet 12 from the extending force to let the outer sheet 12 retract by its own retractability.

The method of the first embodiment will be described in detail. A first continuous web 120 and a second continuous web 130 are provided as a sheet for forming an outer sheet 12 and a sheet for forming an inner sheet 13, respectively. The first continuous web 120 is a stretch sheet of continuous length having stretchability in its longitudinal direction. The second continuous web 130 is a non-stretch sheet of continuous length having substantially no extensibility in its longitudinal direction.

The first continuous web 120 may be a longitudinally stretchable sheet of continuous length that has been prepared separately from the diaper production line. The method of the present embodiment illustrated in Fig. 6, however, includes the step of providing the first continuous web 120 with stretchability developed by processing a non-stretch continuous web 120' or the step of providing the first continuous web 120 with improved stretchability by processing a low-stretch continuous web 120' before the step of extending the first continuous web 120 in the longitudinal direction. In the present embodiment, the thus obtained first continuous web 120 is extended.

The step for developing or improving stretchability included in the first embodiment is an in-line processing step using a pair of intermeshing toothed rollers 71 as shown in Fig. 6. More specifically, the step is carried out by introducing a continuous web 120' composed of an elastic layer (e.g., an elastic fiber layer, an elastic net sheet or an elastic film) and an inelastic fiber layer on one or both sides of the elastic layer into the nip between the toothed rollers 71, whereby the inelastic fiber layer is cut, the constituent fibers of the inelastic fiber layer are extended, or the interfiber fusion bonds in the inelastic fiber layer are destroyed. As a result, the inelastic fiber layer is converted into a structure that hardly impedes the stretch of the elastic layer. By this conversion, there is obtained the first continuous web 120 with developed or improved stretchability.

By providing the step of developing or improving stretchability by in-line processing a non-stretch or low-stretch continuous web 120' before the step of extending the first continuous web 120 in the longitudinal direction, there is obtained a continuous web having equal longitudinal stretchability to that of a stretchable web previously prepared in a line separately from the diaper production line. The width of the non-stretch or low-stretch continuous web 120' is preferably 100% to 180%, more preferably 120% to 160%, of the first continuous web 120, taking the lateral shrinkage in the in-line processing into consideration.

The thus provided first continuous web 120 is continuously fed and extended between a first pair of nip rollers 72 and a second pair of nip rollers 73. The second pair of nip rollers 73 are driven at a higher rotating speed (peripheral speed or web running speed) than the first pair of nip rollers 72. The thus extended first continuous web 120 is passed through a pair of rollers 75a whereby the travelling direction of the web 120 is changed so as to be joined to the second continuous web 130.

The first continuous web 120 (outer sheet 12) in a state stretched in the diaper lateral direction is joined and bonded to the second continuous sheet 130 (inner sheet 13) in a state flattened in the diaper lateral direction. The second continuous web 130 is a non-stretch sheet, and the first continuous web 120 is a stretch sheet. That is, the second continuous web 130 in a substantially non-extended state and the first continuous web 120 in a state having developed contractibility in the diaper lateral direction are bonded to each other.

In the first embodiment, the first continuous web 120 and the second continuous web 130 are joined together with leg elastic members 61a and 61b and waist elastic members 51 sandwiched therebetween as illustrated in Fig. 6. Before the joining, an adhesive 52 and 62 for fixing the elastic members is applied to prescribed positions on either one or both of the continuous webs 120 and 130. The two webs 120 and 130 with the elastic members 61a, 61b, and 51 disposed therebetween in their stretched state are pressed between a pair of nip rollers 74. The two webs 120 and 130 are thus joined while fixing the elastic members 61a, 61b, and 51.

The leg elastic members 61a and 61b are introduced into between the continuous webs 120 and 130 while being rocked across the moving webs 120 and 130 by known rocking guides. The moving direction of the continuous web 130 is changed as prescribed by passage over a pair of guide rollers 75b.

As a result of joining the first continuous web 120 and the second continuous web 130, a continuous-form outer cover 110 having controlled stretchability in its longitudinal direction is obtained. A pair of rollers 75c is provided to change the travelling direction of the continuous-form outer cover 110 as prescribed.

In the first embodiment, the first continuous web 120 is bonded to the second continuous web 130 in its longitudinally stretched state and with a reduced width, i.e., in a crosswise contracted state. Accordingly, it is preferred that the first continuous web 120 before being longitudinally stretched have a width (width in a non-stretched state) corresponding to 100% to 140%, more preferably 110% to 130%, of the width after being joined to the second continuous web 130 so as not to become narrower than the second continuous web 130 due to the crosswise contraction.

The stretch ratio of the first continuous web 120 being joined to the second continuous web 130 is preferably, for example, 1.3 to 4.0 times, more preferably 1.5 to 3.0 times.

The disposable pull-on diaper 1 of the first embodiment is produced in a usual manner for the production of disposable pull-on diapers in a transverse feed system, except for using the thus prepared continuous-form outer cover 110. For example, as shown in Fig. 6, a continuous-form absorbent body 101 is cut into individual absorbent bodies 10, which are turned 90° and spacedly fixed on the continuous-form outer cover 110. The unnecessary part 111 is cut out of the continuous-form outer cover 110 with a rotary cutter, a laser cutter, etc. to make a continuous-form diaper 100.

The absorbent body 10 is fixed to the continuous-form outer cover 110 while maintaining the first continuous web 120 in the stretched state. In other words, the absorbent body 10 is fixed while holding the continuous-form outer cover 110 not to contract due to the retractability of the first continuous web 120.

Both edge portions 12a and 12b of the continuous-form outer cover 110 of the continuous-form diaper 100 are folded over the longitudinal end portions of the individual absorbent bodies 10 and secured thereto. The continuous-form diaper 100 is then folded in two.

The folded continuous-form diaper 100 is spacedly sealed to form side seals S by heat sealing, ultrasonic sealing, high frequency sealing or like means, and the continuous-form diaper 100 is cut into individual disposable pull-on diapers 1 after or simultaneously with the formation of the side seals S.

Ninety-degree turning the absorbent body 10 and spacedly securing the absorbent bodies 10 to the continuous-form outer cover 110 are carried out by, for example, the method taught in JP 4-166150A. A joint region (not shown) for fixing the absorbent body 10 to the continuous outer cover 110 is provided on either one or both of the absorbent body 10 and the continuous outer cover 110. Removal of the unnecessary part 111 for making the leg openings may be carried out before fixing the absorbent body 10 to the continuous outer cover 110.

According to the method of the first embodiment, disposable pull-on diapers having good stretchability in the lateral direction (the horizontal direction in Fig. 2) can be produced stably in a continuous manner with high finish accuracy by using a continuous web having longitudinal stretchability (the first continuous web 120) as described above.

The continuous-form outer cover 110 obtained by joining the first continuous web 120, which has high longitudinal stretchability, to the substantially inextensible second continuous web 130 hardly extends any further or, extends slightly but suddenly stops extending further. Therefore, as long as the continuous-form outer cover 110 is placed under at least a certain tension in the moving direction, the stretch ratio of the continuous-form outer cover 110 hardly varies even if the tension applied to the continuous-form outer cover 110 fluctuates during the movement.

As a result, disposable pull-on diapers with high finish accuracy can be produced in a stable manner because positional accuracy can be secured in (a) placing and bonding the individual absorbent bodies 10 to the continuous-form outer cover 110, (b) punching leg holes 112 through the continuous-form outer cover 110 before or after fixing the individual absorbent bodies 10, (c) folding in two the continuous-form outer cover 110 having the absorbent bodies 10 fixed thereto (i.e., the continuous-form diaper 100), (d) sealing the continuous-form outer cover 110 having the absorbent bodies 10 fixed thereto (i.e., the continuous-form diaper 100) at a given interval to form side seals S, and (e) cutting the continuous-form diaper 100 into individual diapers.

Since the bonding between the first continuous web 120 and the second continuous web 130 is in parts, the stretchability of the continuous-form outer cover 110 owing to the stretchability of the first continuous web 120 is not impaired by the adhesive applied, or the impairment, if any, is very slight. Thus, the resulting disposable diaper 1 is well extensible and retractable in the lateral direction (the horizontal direction in Fig. 2) owing to the stretchability of the first continuous web 120, thereby providing a snug fit against the wearer's body under the waist, pleasant comfort, and high leak-proofness.

In the first embodiment of the production method illustrated in Fig. 6, the first continuous web 120 for forming the outer sheet 12 after having been made longitudinally stretchable is joined as being of continuous length to the second continuous web 130. In a modified embodiment of the method of the invention, it is possible that a first continuous web 120 is processed to develop stretchability in its lateral direction and then cut into sheets. The cut sheets are turned 90° and connected to one another to make a sheet of continuous length having stretchability in its longitudinal direction, namely, machine direction, which is then joined to the second continuous web 130.

Ninety-degree turning a cut sheet can be carried out using a known method or mechanism. Connecting the cut sheets into a continuous form can be conducted by successively arraying the cut sheets in the machine direction with an overlap between every adjoining cut sheets, and the overlaps are united by, for example, heat sealing, ultrasonic sealing, or high frequency sealing.

In the modified embodiment, too, the first continuous web is stretched between the first nip rollers 72 and the second nip rollers 73 and joined to the second continuous web 130 in the stretched state. By using the thus obtained continuous-form outer cover 110, disposable pull-on diapers are produced stably and continuously similarly to the first embodiment shown in Fig. 6.

The disposable pull-on diaper of the present invention will further be illustrated with reference to its second embodiment by way of the accompanying drawing. Unless otherwise described, the description of the first embodiment applies to the second embodiment.

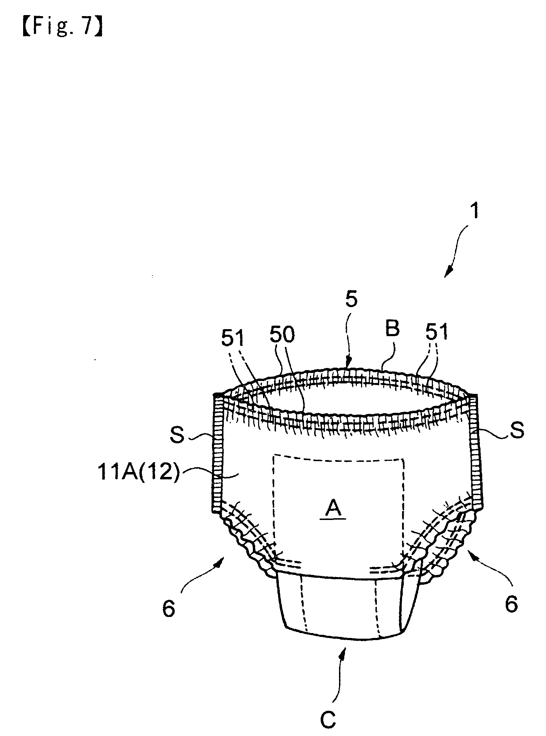

As illustrated in Figs. 7 through 10, the disposable diaper 1 of the second embodiment includes a liquid permeable topsheet 2, a liquid impermeable or water repellent backsheet 3, a substantially oblong absorbent body 10 interposed between the topsheet 2 and the backsheet 3, and an outer cover 11 joined to the side of the backsheet 3 (i.e., the garment facing side) of the absorbent body 10. The absorbent body 10 has a liquid retentive absorbent core 4.

With the diaper 1 in an unfolded state, the outer cover 11 includes a front outer cover portion 11A disposed in the front portion A (located on one of the longitudinal end portions of the absorbent body 10) and a rear outer cover portion 11B disposed in the rear portion B (located on the other longitudinal end portion of the absorbent body 10). The front outer cover portion 11A and the rear outer cover portion 11B have their respective shapes generally corresponding to the front body portion and the back body portion of a pair of panties. Between the front portion A and the rear portion B of the absorbent body 10, namely in the longitudinally middle portion of the absorbent body 10, is there the crotch portion C.

Both side edge portions A1 and A2 of the front outer cover portion 11A and both side edge portion B1 and B2 of the rear outer cover portion 11B are joined to each other to make side seals S. The disposable diaper 1 thus has the shape of a pair of pants with a waist opening 5 and a pair of leg openings 6. Joining the side edge portions is carried out by heat sealing, high frequency sealing, ultrasonic sealing, or like means.

The diaper 1 of the second embodiment has the outer cover only in the front portion A and the rear portion B but not in the crotch portion C. So configured, the diaper 1 has no other members than the absorbent body 10, such as a nonwoven fabric as an outer cover and an adhesive for joining the outer cover, in the crotch portion C. This is advantageous in terms of permeability to air and moisture.

When a wearer's leg is inserted into the leg opening 6 of the diaper 1, the leg opening 6 is widened, and the leg elastic members 61a and 61b are stretched, the cuff elastic members 81 are not stretched in the diaper lateral direction because they are formed independently of the leg elastic members 61a and 61b. Therefore, the leg opening 6 is allowed to widen without increasing the width of the crotch portion C. This is advantageous in that a part of the wearer's foot or leg is not likely to catch in the leg opening 6 when the foot is inserted into the leg opening or when the diaper 1 is pulled up. That is, the diaper 1 is easier to put on a wearer.

The topsheet 2, backsheet 3, and absorbent core 4 are rectangle and are united into an oblong absorbent body 10. The absorbent body 10 of the second embodiment is structurally identical to the absorbent body 10 of the first embodiment and will not be redundantly described.

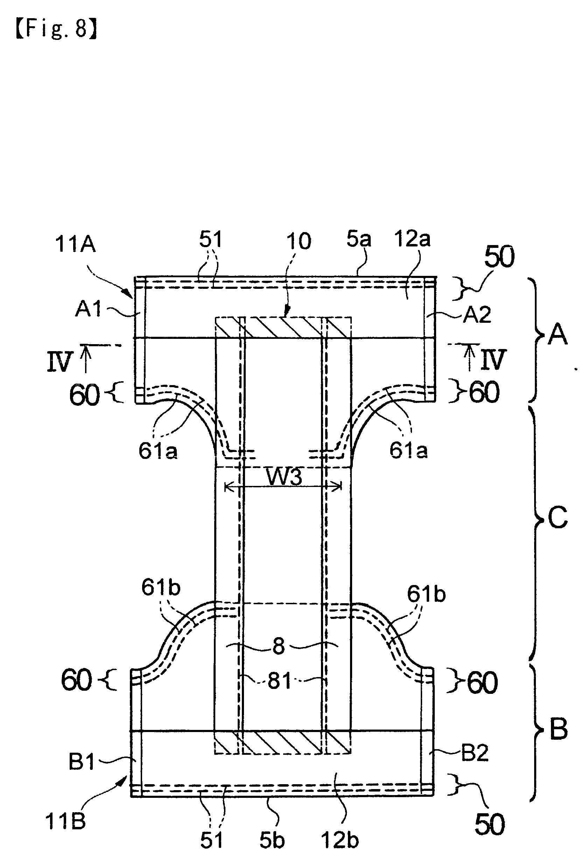

Each of the front outer cover portion 11 A and the back outer cover portion 11B is a laminate composed of a stretch sheet as an outer sheet 12 and a non-stretch sheet as an inner sheet 13. The outer sheet 12 provides the exterior surface of the diaper. The inner sheet 13 is disposed on the inner side of the outer sheet 12. It is only necessary for the stretch sheet forming the outer sheet 12 to be stretchable in at least the lateral direction of the diaper in an unfolded state (the horizontal direction in Fig.8). The non-stretch sheet forming the inner sheet 13 has no stretchability in at least the lateral direction of the diaper in the unfolded state. The details of the outer sheet 12 and the inner sheet 13 will be described later.

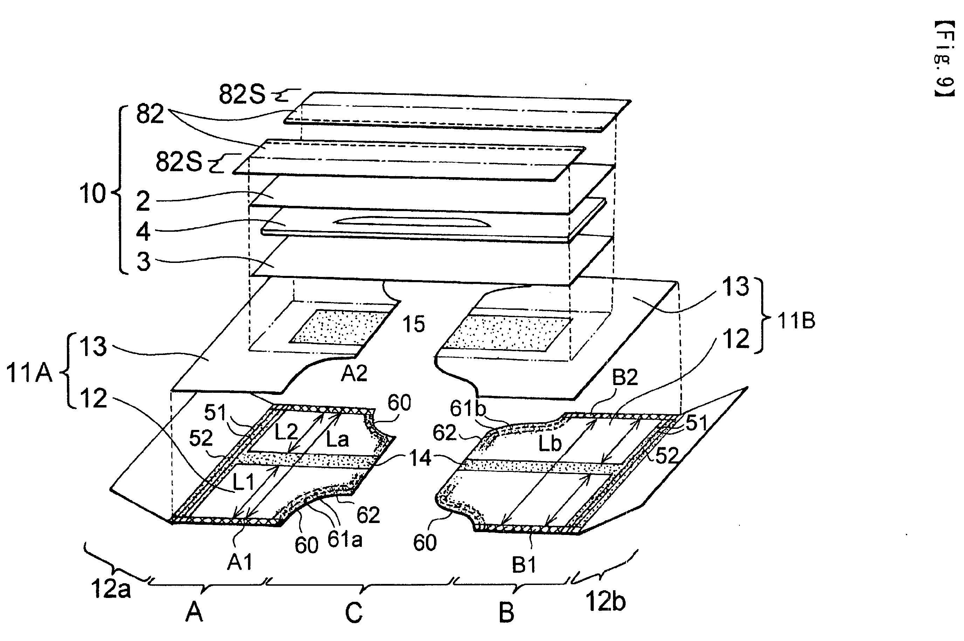

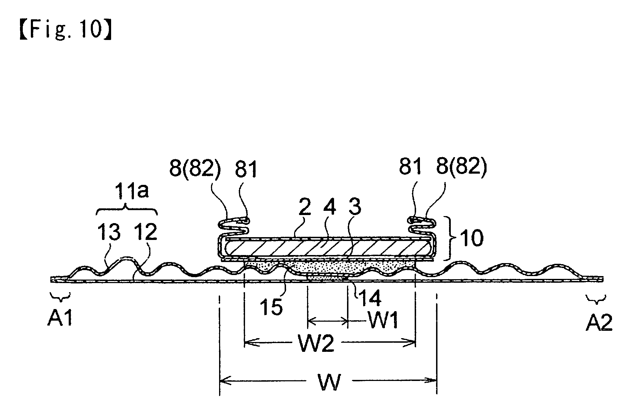

In the disposable diaper 1 of the second embodiment, as illustrated in Figs. 9 and 10, the outer sheet 12 and the inner sheet 13 are joined to each other in the side edge portions A1, A2, B1, and B2 by heat sealing, high frequency sealing or ultrasonic sealing and in the peripheral portion 50 along the waist opening 5 and the peripheral portion 60 along each leg opening 6 by means of an adhesive 52 and 62 (e.g., a hot melt adhesive), respectively. In each of the front outer cover portion 11 A and rear outer cover portion 11B, the outer sheet 12 and the inner sheet 13 are not joined in the most of the area except these portions.

Specifically, the outer sheet 12 and the inner sheet 13 are joined not only in the side edge portions A1, A2, B1, and B2, the peripheral portion 50 along the waist opening 5, and the pair of peripheral portions 60 along the leg openings 6 but also in the laterally middle portion in the front outer cover portion 11A and the rear outer cover portion 11B. The outer sheet 12 and the inner sheet 13 are not joined in other than these portions.

The outer sheet 12 and the inner sheet 13 being not joined over a broad area in the front outer cover portion 11A and the rear outer cover portion 11B, the regions in which the outer cover portions 11A and 11B become stiff due to an adhesive applied can be minimized, and the diaper feels soft and pleasant to the touch on the diaper's exterior surface and the exposed parts (the parts not covered by the absorbent body 10) of the inner surface of the outer cover portions 11 A and 11B.

When the outer sheet 12 and the inner sheet 13 are formed of breathable sheets, there is provided a diaper having good breathability and thereby capable of controlling the inside humidity. The outer sheet 12 and the inner sheet 13 being joined in the peripheral portions 50 and 60 along the waist opening 5 and the leg openings 6, the stretchable outer cover portions 11 A and 11 B provide moderate fit in the portion below the waist opening 5.

The peripheral portion 50 along the waist opening 5 in each of the front outer cover portion 11A and the rear outer cover portion 11 B has a plurality of waist elastic members 51 disposed along the edge of the waist opening 5. The waist elastic members 51 are fixed in their stretched state between the outer sheet 12 and the sinner sheet 13 via the adhesive 52.

The peripheral portion 60 along each of the leg openings 6 in the front outer cover portion 11A and the rear outer cover portion 11B has leg elastic members 61a and 61b disposed along the edge of the leg opening 6. The leg elastic members 61a and 61b are secured between the outer sheet 12 and the inner sheet 13 in their stretched state by the adhesive 62.

The waist elastic members 51 and the leg elastic members 61a and 61b are preferably rubber threads or tapes made from stretch materials, such as natural rubber, polyurethane resins, foamed urethane resins, and hot-melt extensible materials.

With the elastic members 51, 61a, and 61b of thread or tape form being fixed in the peripheral portions 50 and 60 along the waist opening 5 and the leg openings 6 as sandwiched in between the outer sheet 12 and the inner sheet 13, these peripheral portions may be designed to have improved fit without being restricted by the stretch characteristics of the outer cover portions 11 A and 11B.

With the elastic members 51, 61a, and 61b of thread or tape form being fixed as sandwiched in between the outer sheet 12 and the inner sheet 13, there is less likelihood of causing a discomfort to a wearer or spoiling the diaper appearance than when the elastic members are fixed to an outer cover formed of a single sheet.

In particular, the elastic members 61a and 61b provided along the leg openings 6 provide a better fit and prevent the diaper 1 from sliding down. The absence of leg elastic members 61 a and 61b in the crotch portion C makes the diaper 1 easier to put on a wearer and causes no indentations or marks on the wearer's skin.

The part of the leg elastic members 61a and 61 b that is the closest to the crotch portion may be made substantially non-stretchable. In this case, the absorbent body 10 does not reduce in width in the crotch portion, whereby leakage is prevented. The part to be made substantially non-stretchable preferably includes at least the part overlapping the absorbent body 10.

Making a part of the elastic members 61a and 61b substantially non-stretchable can be achieved by, for example, cutting a part of the elastic members 61a and 61b as fixedly sandwiched between the outer sheet 12 and the inner sheet 13 in their stretched state, the part overlapping the absorbent body 10 at a number of points so that the stretchability of that part is no more exerted.

To ensure the above-described effects, it is preferred that the width W3 (see Fig. 8) of the part of the leg elastic members 61a and 61b that is the closest to the crotch portion and has substantially no stretchability be equal to or larger than the width of a joint region via which the absorbent body 10 is bonded to the front outer cover portion 11A and the rear outer cover portion 11B (i.e., the width W2 of a joint region 15 hereinafter described, see Fig. 10).

In the disposable diaper 1 of the second embodiment, the width of the peripheral portion 50 along the waist opening 5 where the outer sheet 12 and the inner sheet 13 are joined is preferably within 70 mm, more preferably within 60mm, from the edges 5a and 5b of the waist opening in the front outer cover portion 11A and the rear outer cover portion 11B, respectively. The width of the peripheral portion 60 along the leg opening 6 where the outer sheet 12 and the inner sheet 13 are joined is preferably within 50 mm, more preferably within 30 mm, from the edge of the leg opening 6 in each of the front outer cover portion 11A and the rear outer cover portion 11 B.

The regions in each of the front outer cover portion 11 A and the rear outer cover portion 11B where the outer sheet 12 and the inner sheet 13 are not joined together preferably have a total length in the diaper lateral direction, L1+L2, of 60% or more, more preferably 70% or more, even more preferably 75% or more, of the length La (or Lb) between the laterally opposing side edge portions A1 and A2.

The region in each of the front outer cover portion 11 A and the rear outer cover portion 11B where the outer sheet 12 and the inner sheet 13 are not joined preferably has a total area of 60% of 100%, more preferably 70% to 100%, of the area of the front portion A or the rear portion B, respectively. When the area of the region where the outer sheet 12 and the inner sheet 13 are not joined falls within the above recited range, the area is referred to as "the entire or almost the entire area".

The dimensions, ratios of dimensions, and the like as referred to in the second embodiment are those measured or calculated on a diaper in a flat-out state with all the elastic members along the waist opening and the leg openings being in a relaxed state with no contractibility (with no outer force, like tension, applied thereto) as illustrated in Fig. 8. The measured values are based on the dimension of the outer sheet in its relaxed state.

In the disposable diaper 1 of the second embodiment, the outer sheet 12 and the inner sheet 13 are joined to each other in the laterally middle portion of each of the front outer cover portion 11 A and the rear outer cover portion 11 B. That is, the two sheets are joined in a joint region 14 extending in the diaper longitudinal direction with a certain width. With the outer sheet 12 and the inner sheet 13 joined in the joint region 14, the diaper has improved appearance both before and during use. In the case where a tape for disposal (not shown) is provided on the exterior surface of the front outer cover portion 11A or the rear outer cover portion 11B, the tape is firmly secured to the laterally middle portion. The tape for disposal is means to hold a used diaper in a rolled condition. Various conventional tapes for disposal can be used.

With the outer sheet 12 and the inner sheet 13 being joined in the joint region 14 in the front outer cover portion 11 A and the rear outer cover portion 11B, a pattern, a letter, or any other design if printed on the backsheet 3 is easily seen from the outside through the outer cover.

The joint region 14 between the outer sheet 12 and the inner sheet 13 in the laterally middle portion of each of the front outer cover portion 11A and the rear outer cover portion 11B preferably has a width W1 of 0 to 40%, more preferably 0 to 30%, of the width W of the absorbent body 10. With the width W1 of the joint region 14 being within 40% of the width W of the absorbent body 10, the stretchability of the outer cover is less likely to be impeded.

As illustrated in Figs. 9 and 10, the part of the absorbent body 10 other than the longitudinal end portions thereof is joined to the inner sheet 13 of each of the front outer cover portion 11A and the rear outer cover portion 11 B in a joint region 15 provided on the laterally middle portion thereof. In view of preventing the absorbent body 10 from separating from the front outer cover portion 11A or breaking during use of the diaper 1, the total area T2 of the joint region between the absorbent body 10 and the front outer cover portion 11A to the area T1 of the front outer cover portion-facing surface of the absorbent body 10, T2/T1, is preferably at least 0.6, more preferably 0.65 or more, with the front outer cover portion 11 A being in a relaxed (contracted) state. Likewise, with the rear outer cover portion 11B in a relaxed state, the total area T2' of the joint region between the absorbent body 10 and the rear outer cover portion 11B to the area T1' of the rear outer cover portion-facing surface of the absorbent body 10, T2'/T1', is preferably at least 0.6, more preferably 0.65 or more. T2/T1 and T2'/T1' may be equal or different. The upper limits of T2/T1 and T2'/T1' are most preferably 1.

The joint region 14 between the outer sheet 12 and the inner sheet 13 in the laterally middle portion of each of the front outer cover portion 11A and the rear outer cover portion 11 B preferably has a width W1 of 0 to 40%, more preferably 0 to 30%, of the width W of the absorbent body 10. With the width W1 of the joint region 14 being within 40% of the width W of the absorbent body 10, the stretchability of the outer cover is less likely to be impeded.

The joint region 15 is a rectangular region extending in the longitudinal direction in the laterally middle portion of the absorbent body 10. The width W2 of the joint region 15 is preferably at least 70%, more preferably 75% or more, of the width W of the garment facing side of the absorbent body 10 in each of the front portion A and the rear portion B. With the width W2 of the joint region 15 being 70% or more of the width W of the absorbent body 10, the absorbent body 10 is less likely to separate or break during wear.

In the second embodiment, when the front outer cover portion 11 A and the rear outer cover portion 11B are in a flat-out state with the side seals S taken apart, the substantial width of the inner sheet 13 is larger than the width of the outer sheet 12 in a relaxed state. When the front outer cover portion 11 A and the rear outer cover portion 11B are in a flat-out state, the outer sheet 12 is in a contracted state due to its retractability, while the inner sheet 13, being formed of a non-stretch sheet, does not virtually reduce in width. As a result, the inner sheet 13 is loose and slack in the lateral direction relative to the outer sheet 12. By the expression "substantial width of the inner sheet 13" as used herein is meant the width of the inner sheet 13 imaginarily separated from the outer sheet 12 and flattened out.

The substantial width of the inner sheet 13 is preferably 1.3 to 4.0 times, more preferably 1.5 to 3.0 times, the width of the outer sheet 12 in its relaxed state. The front outer cover portion 11A and the rear outer cover portion 11 B having such a structure are prepared by, for example, the method described *infra*.

In the so designed disposable pull-on diaper 1 of the second embodiment, the substantial width of the inner sheet 13 is larger than the width of the outer sheet 12 in the relaxed state with the side seals S taken apart, and the front outer cover portion 11A and the rear outer cover portion 11B flattened out. By virtue of this configuration as well as the fact that the outer sheet 12 of the front and rear outer cover portions 11A and 11B is formed of a stretch sheet, the diaper has a neat appearance and a good fit while worn.

Since the inner sheet 13 of the front outer cover portion 11A and the rear outer cover portion 11B is formed of a non-stretch sheet, even when the outer cover portions 11A and 11B are extended in the lateral direction on use, the inner sheet 13 is slackened, and there is less likelihood that the absorbent body 10 separates from the front outer cover portion 11A or the rear outer cover portion 11B and that the outer cover portion 11 A or 11 B breaks. Furthermore, the skin contacting surfaces of the front and rear outer cover portions 11A and 11B have good feel and hand with excellent softness.

The outer sheet 12 formed of a stretch sheet and the inner sheet 13 formed of a non-stretch sheet not being joined to each other in the most part of the front and rear outer cover portions 11A and 11B, the front and rear outer cover portions 11A and 11B exhibits good stretchability with little influence from the non-stretchable absorbent body 10. When the outer sheet 12 of the front and rear outer cover portions 11 A and 11B is released from an extended state, the absorbent body 10 continues having a good appearance and high absorptivity without being bunched up. Therefore, the lateral stretchability of the front and rear outer cover portions 11A and 11B is not impeded even when the absorbent body 10 is designed to have a wide joint region 15.

The outer sheet 12 used in the front and rear outer cover portions 11 A and 11B of the diaper 1 of the second embodiment has stretchability as previously stated.

The outer sheet 12 and the inner sheet 13 used in the second embodiment are structurally equal to those in the first embodiment and will not be described redundantly.

The outer sheet 12 generally has lower sheet strength than the inner sheet 13 so that the front or rear outer cover portion 11A or 11B can tear while worn or be broken through with the finger of a diaperer when in putting the diaper on a wearer. To avoid this, it is more desirable for the inner sheet 13 to have higher strength. Specifically, the inner sheet 13 preferably has a breaking strength of 5 N/25 mm or higher in both the diaper lateral and longitudinal directions. The breaking strength is measured by the method previously described.

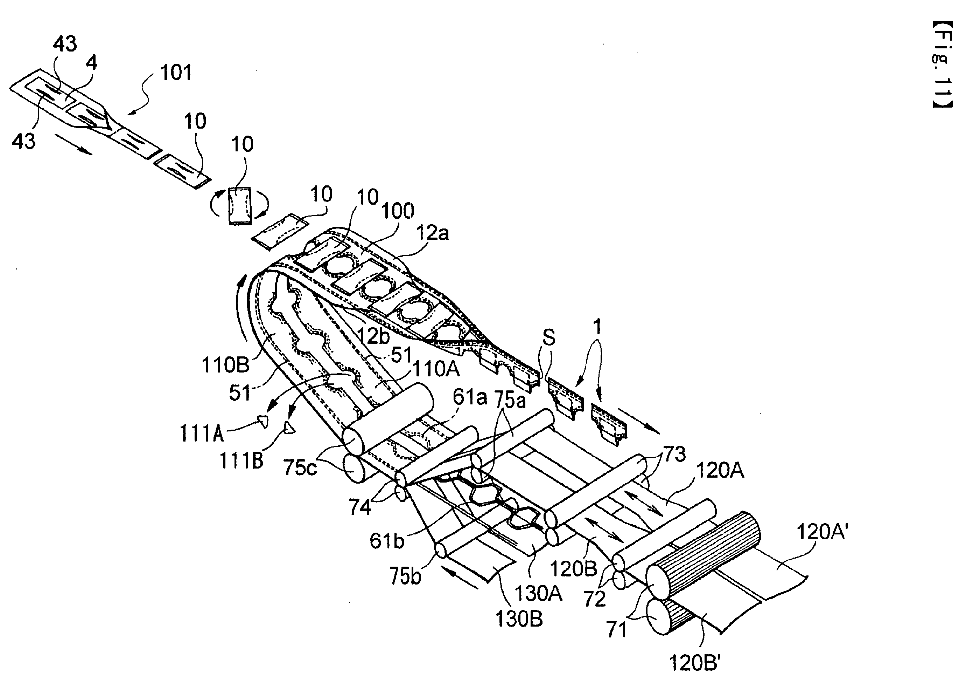

A suitable method for producing the disposable pull-on diaper of the second embodiment will then be described. The method described hereunder is for producing the disposable pull-on diaper 1 of the second embodiment in a continuous manner. As illustrated in Fig. 11, this method includes joining an outer sheet 12 in its laterally extended state to an inner sheet 13 in its laterally flattened state to make a front outer cover portion and a rear outer cover portion, joining an absorbent body 10 to the inner sheet 13 of the two outer cover portions via respective joint regions (not shown) while maintaining the outer sheet 12 of the two outer cover portions in the extended state, and releasing the outer sheet

12 from the extending force to let the outer sheet 12 retract by its own retractability.

The method will be described in more detail. Two first continuous webs 120A and 120B are provided each for forming an outer sheet 12. Two second continuous webs 130A and 130B are provided each for forming an inner sheet 13. The first continuous webs 120A and 120B are each a stretch sheet of continuous length having stretchability in its longitudinal direction. The second continuous webs 130A and 130B are each a non-stretch sheet of continuous length having substantially no extensibility in its longitudinal direction.

The first continuous webs 120A and 120B may be each a longitudinally stretchable sheet of continuous length that has been prepared separately from the diaper production line. The method of the present embodiment, however, includes the step of providing the first continuous webs 120A and 120B with stretchability developed by processing non-stretch continuous webs 120A' and 120B', respectively, or the step of providing the first continuous webs 120A and 120B with improved stretchability by processing low-stretch continuous webs 120A' and 120B', respectively, before the step of extending the first continuous webs 120A and 120B in the longitudinal direction as illustrated in Fig. 11. The thus obtained first continuous webs 120A and 120B are extended.

The step for developing or improving stretchability included in the present embodiment is an in-line processing step using a pair of intermeshing toothed rollers 71 as shown in Fig. 11. More specifically, the step is carried out by introducing continuous webs 120A' and 120B' each composed of an elastic layer (e.g., an elastic fiber layer, an elastic net sheet or an elastic film) and an inelastic fiber layer on one or both sides of the elastic layer into the nip between the toothed rollers 71, whereby the inelastic fiber layer is cut, the constituent fibers of the inelastic fiber layer are extended, or the interfiber fusion bonds in the inelastic fiber layer are destroyed. As a result, the inelastic fiber layer is converted into a structure that hardly impedes the stretch of the elastic layer. By this conversion, there is obtained the first continuous webs 120A and 120B with developed or improved stretchability.

By providing the step of developing or improving stretchability by in-line processing non-stretch or low-stretch continuous webs 120A' and 120B' before the step of extending the first continuous webs 120A and 120B in the longitudinal direction, there is obtained continuous webs having equal longitudinal stretchability to that of those previously prepared in a line separately from the diaper production line. The width of the non-stretch or low-stretch continuous webs 120A' and 120B' is preferably 100% to 180%, more preferably 120% to 160%, of the first continuous webs 120A and 120B, respectively, taking the lateral shrinkage in the in-line processing into consideration.

The thus provided first continuous webs 120A and 120B are continuously fed and extended between a first pair of nip rollers 72 and a second pair of nip rollers 73. The second pair of nip rollers 73 are driven at a higher rotating speed (peripheral speed or web running speed) than the first pair of nip rollers 72. The thus extended first continuous webs 120A and 120B are passed through a pair of rollers 75a whereby the travelling direction of these webs is changed so as to be joined to the second continuous webs 130A and 130B.

The first continuous webs 120A and 120B (outer sheets 12) in a state stretched in the diaper lateral direction are joined and bonded to the second continuous sheets 130A and 130B (inner sheets 13), respectively, in a state flattened in the diaper lateral direction. The second continuous webs 130A and 130B are each a non-stretch sheet, and the first continuous webs 120A and 120B are each a stretch sheet. That is, the second continuous webs 130A and 130B in a substantially non-extended state and the first continuous webs 120A and 120B in a state having developed contractibility in the diaper lateral direction are bonded to each other.

In the present embodiment, the first continuous webs 120A and 120B and the second continuous webs 130A and 130B are joined together, respectively, with leg elastic members 61a and waist elastic members 51 sandwiched between the first continuous web 120A and the second continuous web 130A and with leg elastic members 61b and waist elastic members 51 sandwiched between the first continuous web 120B and the second continuous web 130B as illustrated in Fig. 11. Before the joining, an adhesive 52 and 62 for fixing the elastic members is applied to prescribed positions on either one or both of the first continuous web 120A and the second continuous web 130A. The two webs 120A and 130A with the elastic members 61a and 51 disposed therebetween in their stretched state are pressed between a pair of nip rollers 74. The two webs 120A and 130A are thus joined while fixing the elastic members 61 a and 51. The first continuous web 120B and the second continuous web 130B are joined in exactly the same manner.

The leg elastic members 61a and 61b are introduced into between the continuous webs 120A and 130A and between the continuous webs 120B and 130B, respectively, while being rocked across the moving webs 120A, 120B, 130A, and 130B by known rocking guides. The traveling direction of the continuous webs 130A and 130B is changed as prescribed by passage over a guide roller 75b.

As a result of joining the first continuous webs 120A and 120B and the second continuous webs 130A and 130B, respectively, a continuous-form front outer cover portion 110A and a continuous-form rear outer cover portion 110B both having controlled stretchability in their longitudinal direction are obtained. A pair of rollers 75c is provided to change the moving direction of the continuous-form front outer cover portion 110A and the continuous-form rear outer cover portion 110B as prescribed.

In the present embodiment, the first continuous webs 120A and 120B are bonded, in their longitudinally stretched state and with a reduced width, i.e., in a crosswise contracted state, to the second continuous webs 130A and 130B.

Accordingly, it is preferred that the first continuous webs 120A and 120B before being longitudinally stretched have a width (width in a non-stretched state) of 100% to 140%, more preferably 110% to 130%, of the width after being joined to the second continuous webs 130A and 130B so as not to become narrower than the second continuous webs 130A and 130B due to the crosswise contraction. The stretch ratio of the first continuous webs 120A and 120B being joined to the second continuous webs 130A and 130B is preferably, for example, 1.3 to 4.0 times, more preferably 1.5 to 3.0 times.

The disposable pull-on diaper 1 of the second embodiment is produced in a usual manner for the production of disposable pull-on diapers in a transverse feed system, except for using the thus prepared continuous-form front outer cover portion 110A and continuous-form rear outer cover portion 110B. For example, as shown in Fig. 11, a continuous-form absorbent body 101 is cut into individual absorbent bodies 10, which are turned 90° and spacedly fixed on the continuous-form front outer cover portion 110A and the continuous-form rear outer cover portion 110B. The unnecessary semicircular parts 111A and 111B are cut out of each continuous-form outer cover portion with a rotary cutter, a laser cutter, etc. to make a continuous-form diaper 100.

The absorbent body 10 is fixed to the continuous-form front and rear outer cover portions 110A and 110B while maintaining the first continuous webs 120A and 120B in their stretched state. In other words, the absorbent body 10 is fixed while holding the continuous-form front and rear outer cover portions 110A and 110B not to contract due to the retractability of the first continuous webs 120A and 120B.

Edge portions 12a and 12b of the continuous-form front outer cover portion 110A and the continuous-form rear outer cover portion 110B, respectively, of the continuous-form diaper 100 are folded over the longitudinal end portions of the individual absorbent bodies 10 and secured thereto. The continuous-form diaper 100 is then folded in two.

The folded continuous-form diaper 100 is spacedly sealed to form side seals S by heat sealing, ultrasonic sealing, high frequency sealing or like means and cut into individual disposable pull-on diapers 1 either after or simultaneously with the formation of the side seals S.

Ninety-degree turning the absorbent body 10 and spacedly securing the absorbent bodies 10 to the continuous-form front and rear outer cover portions 110A and 110B are carried out by, for example, the method taught in JP 4-166150A. Joint regions (not shown) for fixing the absorbent body 10 to the continuous front and rear outer cover portions 110A and 110B are provided on either one or both of the absorbent body 10 and the continuous front and rear outer cover portions 110A and 110B.

According to the above described method of production, disposable pull-on diapers having good stretchability in the lateral direction (the horizontal direction in Fig. 8) can be produced stably and continuously with high finish accuracy by using continuous webs having longitudinal stretchability (the first continuous webs 120A and 120B) as described above.

The continuous-form front and rear outer cover portions 110A and 110B obtained by joining the first continuous webs 120A and 120B, which have high longitudinal stretchability, to the substantially inextensible second continuous webs 130A and 130B, respectively, hardly extend any further or, extend slightly but suddenly stop extending further. Therefore, as long as the continuous-form front and rear outer cover portions 110A and 110B are placed under at least a certain tension in the moving direction, the stretch ratio of the continuous-form front and rear outer cover portions 110A and 110B hardly varies even if the tension applied thereto fluctuates during the movement.

As a result, disposable pull-on diapers with high finish accuracy can be produced in a stable manner because positional accuracy can be secured in (a) cutting off unnecessary parts 111 A and 111B from the continuous-form front and rear outer cover portions 110A and 110B, respectively, (b) placing and bonding the individual absorbent bodies 10 to the continuous-form front and rear outer cover portions 110A and 110B, (c) folding in two the continuous-form diaper 100, (d) sealing the continuous-form front and rear outer cover portions 110A and 110B having the absorbent bodies 10 fixed thereto at a given interval to form side seals S, and (e) cutting the continuous-form diaper 100 into individual diapers.

Since the bonding between the first continuous web 120A and the second continuous web 130A and the bonding between the first continuous web 120B and the second continuous web 130B are in parts, the stretchability of the resulting continuous-form front and rear outer cover portions 110A and 110B owing to the stretchability of the first continuous webs 120A and 120B is not impaired by the adhesive applied, or the impairment, if any, is very slight. Thus, the resulting disposable diaper 1 is well extensible and retractable in the lateral direction (the horizontal direction in Fig. 8) owing to the stretchability of the first continuous webs 120A and 120B, thereby providing a snug fit against the wearer's body under the waist, pleasant comfort, and high leak-proofness.

While, in the production method illustrated in Fig. 11, the first continuous webs 120A and 120B for forming the outer sheet 12 after having been made longitudinally stretchable are joined as being of continuous length to the second continuous webs 130A and 130B for forming the inner sheet 13, respectively, another way of joining may be adapted. For instance, it is possible that each of the first continuous webs 120A and 120B is processed to develop stretchability in its lateral direction and then cut into sheets at intervals. The cut sheets are turned 90° and connected to one another to make a sheet of continuous length having stretchability in its longitudinal direction, namely, machine direction, which is then joined to the second continuous webs 130A and 130B.

Ninety-degree turning a cut sheet can be carried out using a known method or mechanism. Connecting the cut sheets into a continuous form can be conducted by successively arraying the cut sheets in the machine direction with an overlap between every adjoining cut sheets, and the overlaps are united by, for example, heat sealing, ultrasonic sealing, or high frequency sealing.

In the present method, too, the first continuous webs are stretched between the first nip rollers 72 and the second nip rollers 73 and joined to the second continuous webs in the stretched state. By using the thus obtained continuous-form front and rear outer cover portions, disposable pull-on diapers are produced stably and continuously similarly to the embodiment shown in Fig. 11.

While the invention has been described with respect to its preferred embodiments, it should be understood that the invention is not construed as being limited thereto.

The form of the joint region 14, which is the joint between the outer sheet 12 and the inner sheet 13 in addition to the side seals S and the peripheral portions 50 and 60 around the waist opening 5 and the leg openings 6, is not limited to that used in the first and second embodiments (the elongated rectangle extending over the whole length in the lateral middle).

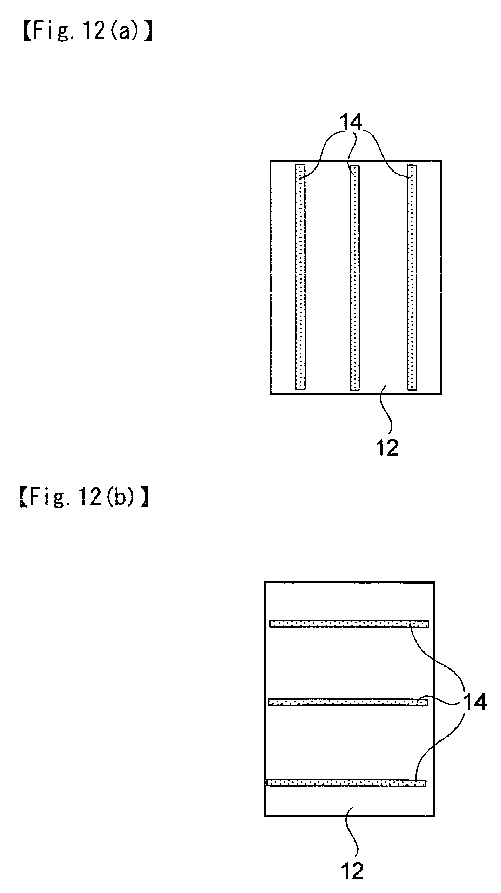

For example, a plurality of joint regions 14 may be laterally spaced. Such an arrangement is exemplified by laterally spaced strip-shaped joint regions 14 each extending in the longitudinal direction as illustrated in Fig. 12(a). A plurality of joint regions 14 may be longitudinally spaced as exemplified by the arrangement of Fig. 12(b) in which strip-shaped joint regions 14 each extending in the lateral direction are longitudinally spaced. The laterally extending strip-shaped joint region 14 may be provided only in the longitudinal middle or only in the longitudinal end portions.

The joint region 14 may be dispensed with.

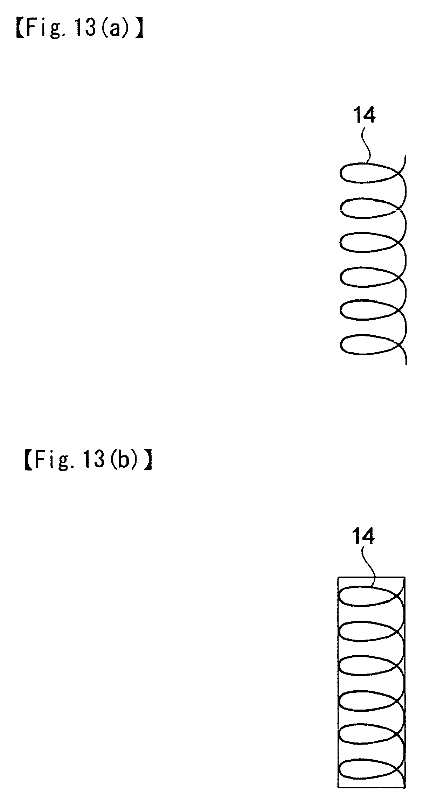

The joint region 14 may have other than an elongated rectangular shape, such as a spiral shape as illustrated in Fig. 13(a). In the case when the joint region 14 has other than an elongated rectangular shape, the minimum area of an imaginary rectangle circumscribing the joint region 14 as drawn in Fig. 13(b) is taken as the joint area of the joint region. In the case where the joint region 14 has other than an elongated rectangular shape, too, the area of the region in each of the front portion A and the rear portion B where the outer sheet 12 and the inner sheet 13 are not joined is preferably 60% to 100%, more preferably 70% to 100%, of the area of the front portion A or the rear portion B.

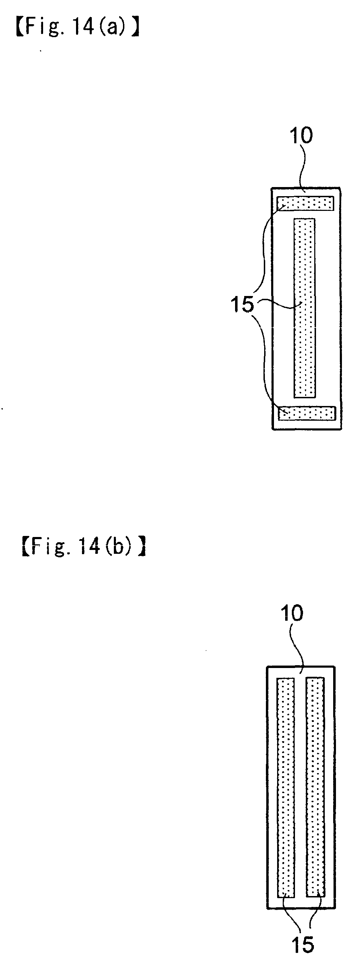

The joint region 15 is not limited to the shape used in the first and second embodiments. Figs. 14(a) and 14(b) are each a bottom view of the absorbent body 10 (seen from the side facing the outer cover 11) showing another application shape of the joint region 15. The shape of the joint region 15 illustrated in Fig. 14(a) is a combination of a longitudinally extending strip in the lateral middle and a laterally extending strip in both longitudinal ends of the absorbent body 10, representing the shape similar to the letter I as a whole. The joint region 15 may be discrete in the lateral direction as exemplified by the arrangement of Fig. 14(b), in which the joint region 15 may be a combination of two laterally spaced strips extending in the longitudinal direction.

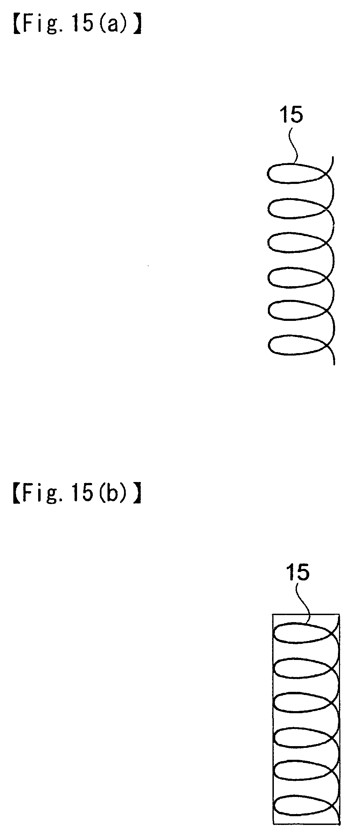

The joint region 15 may have other than a rectangular shape, such as a spiral shape as illustrated in Fig. 15(a). In the case when the joint region 15 has other than a rectangular shape, the minimum area of an imaginary rectangle circumscribing the joint region 15 as drawn in Fig. 15(b) is taken as the joint area of the joint region. In the first embodiment, when the joint region 15 has other than a rectangular shape, too, the total area of the joint region 15 to the area T1 of the outer cover-facing surface of the absorbent body 10, T2/T1, is preferably at least 60%, more preferably 65% or more.

In the second embodiment, when the joint region 15 has other than a rectangular shape, too, the total area T2 of the joint region between the absorbent body 10 and the front outer cover portion 11A to the area T1 of the front outer cover portion-facing surface of the absorbent body 10, T2/T1, is preferably at least 0.6, more preferably 0.65 or more, with the front outer cover portion 11A being in a relaxed state. Likewise, with the rear outer cover portion 11B in a relaxed state, the total area T2' of the joint region between the absorbent body 10 and the rear outer cover portion 11B to the area T1' of the rear outer cover portion-facing surface of the absorbent body 10, T2'/T1', is preferably at least 0.6, more preferably 0.65 or more.

Means for providing the joint region 14 between the outer sheet 12 and the inner sheet 13 and the joint region 15 between the absorbent body 10 and the outer cover 11 is not limited to applying an adhesive and may be general thermal means such as embossing and ultrasonic sealing.

The positions of joining the both side edge portions A1 and A2 of the front portion A and the both side edge portions B1 and B2 of the rear portion B do not need to be right on the sides of a wearer and may be slightly off the sides to either the front or the back.

The disposable diaper 1 of the first embodiment may be designed to be rolled up into a compact form for disposal while retaining its stretch characteristics by attaching a tape for disposal to the garment facing side (the side of the outer sheet 12) of the outer cover 11 at a position overlapping the joint region 14 (e.g., a hot melt adhesive-applied region).

While in the second embodiment the outer cover joined to the garment facing side of the absorbent body is composed of two portions, the front outer cover portion and the rear outer cover portion, the diaper may additionally have a crotch outer cover portion disposed on the crotch portion of the absorbent member, which is located between the front outer cover portion and the rear outer cover portion. The crotch outer cover portion may be united to the front and the rear outer cover portions.

In the second embodiment, the positions of joining the both side edge portions A1 and A2 of the front portion A and the both side edge portions B1 and B2 of the rear portion B do not need to be right on the sides of a wearer and may be slightly off the sides to either the front or the back.

The disposable diaper 1 of the second embodiment may be designed to be rolled up into a compact form for disposal while retaining its stretch characteristics by attaching a tape for disposal to the garment facing side (the side of the outer sheet 12) of either the front outer cover portion 11A or the rear outer cover portion 11B at a position overlapping the joint region 14 (e.g., a hot melt adhesive-applied region).

In the case where a crotch outer cover portion is provided between the front and rear outer cover portions, a leg elastic member may be provided in both lateral side portions of the crotch outer cover portion, and the lateral side portions may be folded back to secure the leg elastic members to form leg gathers. Furthermore, a standing gather may be provided on both sides of the crotch outer cover portion.

The structures and configurations featuring the aforementioned embodiments are appropriately combined with one another.

Industrial Applicability

The disposable pull-on diaper according to the invention has a neat appearance, provides a good fit during wear, is easy to put on a wearer, and is free from such inconveniences as separation of the absorbent body from the outer cover and breaking of the outer cover.

The above-described disposable pull-on diaper is produced efficiently by the production method according to the invention.

**Claims**

1. A disposable pull-on diaper (1) comprising an absorbent body (10) containing an absorbent core (4) and an outer cover (11) joined to the garment facing side of the absorbent body, the outer cover (11) comprising a front portion (A) and a rear portion (B) and having both side edge portions (A1, A2) of the front portion (A) and both side edge portions (B1, B2) of the rear portion (B) jointed together to form a pair of side seals (S), a waist opening (5), and a pair of leg openings (6),
   the outer cover (11) being a laminate comprising an outer sheet (12) formed of a stretch sheet and an inner sheet (13) formed of a non-stretch sheet,
   the outer sheet (12) and the inner sheet (13) being joined to each other in the side seals (A1, A2, B1, B2), a peripheral portion (50) along the waist opening (5), and a peripheral portion (60) along each of the leg openings (6) and along a laterally middle portion (14) in the front portion (A), the rear portion (B), and a crotch portion (C) of the outer cover (11), and not joined to each other in the entire area of the outer cover except the side seals, the peripheral portions and said laterally middle portion in the front portion, the rear portion, and the crotch portion of the outer cover,
   wherein the outer sheet (12) provides an exterior surface of the diaper, and the inner sheet (13) is disposed on the inner side of the outer sheet;
   the diaper further comprising a tape for disposal provided on the exterior surface of the outer cover (11), the tape being firmly secured to the laterally middle portion (14).

2. The disposable pull-on diaper according to claim 1, wherein the inner sheet (13) has a substantial width 1.3 to 4.0 times the width of the outer sheet (12) with the side seals taken apart, the pair of side seals unfolded, and the outer sheet (12) relaxed.

3. The disposable pull-on diaper according to claim 1 or 2, wherein each of the outer sheet (12) and the inner sheet (13) is formed of nonwoven fabric made of thermoplastic fibers.

4. The disposable pull-on diaper according to any one of claims 1 to 3, wherein the outer sheet (12) is a laminate sheet having air-through nonwoven fabric on at least the garment facing side thereof.

5. The disposable pull-on diaper according to any one of claims 1 to 4, wherein the total area T2 of the joint region to the area T1 of the outer cover-facing surface of the absorbent body (10), T2/T1, is at least 60%, with the outer cover (11) in a relaxed state.

6. The disposable pull-on diaper according to any one of claims 1 to 5, wherein the absorbent body (10) and the inner sheet (13) of the outer cover (11) being joined together via a joint region.

7. A disposable pull-on diaper comprising an absorbent body (10) containing an absorbent core (4) and an outer cover (11) joined to the garment facing side of the absorbent body, the outer cover (11) comprising a front portion (A) and a rear portion (B), wherein the outer cover (11) comprises a front outer cover portion (11A) and a rear outer cover portion (11B),
the front outer cover portion (11A) and the rear outer cover portion (11B) being joined to each other along both side edge portions (A1, A2; B1, B2) thereof to form a pair of side seals (S), a waist opening (5), and a pair of leg openings (6), each of the front outer cover portion (11A) and the rear outer cover portion (11B) being a laminate composed of an outer sheet (12) formed of a stretch sheet and an inner sheet (13) formed of a non-stretch sheet, and
the outer sheet (12) and the inner sheet (13) being joined to each other in the side seals (A1, A2; B1, B2), a peripheral portion (50) along the waist opening (5), and a peripheral portion (60) along each of the leg openings (6) and laterally middle portion (14) of each of the front outer cover portion (11A) and the rear outer cover portion (11B), and not joined to each other in the entire area of the outer cover (11) except the side seals (A11, A2; B1, B2), the peripheral portions (50, 60) and said laterally middle portion in the front portion, and the rear portion of the outer cover, the diaper further comprising a tape for disposal provided on the exterior surface of the front outer cover portion (11A) or the rear outer cover portion (11B), the tape being firmly secured to the laterally middle portion (14).

8. The disposable pull-on diaper according to claim 6, wherein each of the front outer cover portion (11A) and the rear outer cover portion (11B) is joined to the absorbent body (10) via the respective joint regions, the joint regions each having a rectangular shape extending in the longitudinal direction of the absorbent body (10).

9. A method of producing a disposable pull-on diaper comprising an absorbent body (10) containing an absorbent core (4) and an outer cover (11) joined to the garment facing side of the absorbent body (10), the outer cover (11) comprising a front portion (A) and a rear portion (B) and having both side edge portions (A1, A2) of the front portion (A) and both side edge portions (B1, B2) of the rear portion (B) jointed together to form a pair of side seals (S), a waist opening (5), and a pair of leg openings (6), the outer cover (11) being a laminate composed of an outer sheet (12) formed of a stretch sheet and an inner sheet (13) formed of a non-stretch sheet, the outer sheet (12) and the inner sheet (13) being joined to each other in the side seals (S), a peripheral portion (50) along the waist opening (5), and a peripheral portion (60) along each of the leg openings (6) and along a laterally middle portion (14) in the front portion (A), the rear portion (B), and a crotch portion (C) of the outer cover (11), and not joined to each other in the entire area of the outer cover except the side seals, the peripheral portions and said laterally middle portion in the front portion, the rear portion, and the crotch portion of the outer cover, and the inner sheet (13) having a substantial width larger than the width of the outer sheet (12) with the side seals taken apart, the pair of side seals unfolded, and the outer sheet (12) relaxed,
the method comprising the steps of
joining the outer sheet (12) in a laterally extended state to the inner sheet (13) in a laterally flattened state;
joining the absorbent body (10) to the inner sheet (13) while maintaining the outer sheet (12) in the extended state, releasing the outer sheet (12) from the extending force to have the outer sheet (12) retract by its own retractability, the outer sheet (12) providing an exterior surface of the diaper, and the inner sheet (13) being disposed on the inner side of the outer sheet; and
providing a tape for disposal provided on the exterior surface of the outer cover or the rear portion of the outer cover (11), the tape being firmly secured to the laterally middle portion (14).

**Patentansprüche**

1. Einweg-Höschenwindel (1), die einen absorbierenden Körper (10), der einen absorbierenden Kern (4) enthält, und eine Außenhülle (11) aufweist, die mit der kleidungszugewandten Seite des absorbierenden Körpers verbunden ist, wobei die Außenhülle (11) einen vorderen Abschnitt (A) und einen hinteren Abschnitt (B) aufweist und beide Seitenkantenabschnitte (A1,A2) des vorderen Abschnitts (A) und beide Seitenkantenabschnitte (B1,B2) des hinteren Abschnitts (B) miteinander verbunden sind, um ein Paar Seitenversiegelungen (S), eine Taillenöffnung (5) und ein Paar Beinöffnungen (6) zu bilden,
wobei die Außenhülle (11) ein Laminat ist, das eine Außenschicht (12), die aus einer dehnbaren Schicht gebildet ist, und eine Innenschicht (13), die aus einer nicht-dehnbaren Schicht gebildet ist, aufweist,
wobei die Außenschicht (12) und die Innenschicht (13) miteinander verbunden sind in den Seitenversiegelungen (A1,A2,B1,B2), einem Randabschnitt (50) entlang der Taillenöffnung (5) und einem Randabschnitt (60) entlang jeder der Beinöffnungen (6) und entlang eines in Querrichtung mittleren Abschnitts (14) in dem vorderen Abschnitt (A), dem hinteren Abschnitt (B) und einem Schrittabschnitt (C) der Außenhülle (11) und nicht miteinander verbunden sind in der gesamten Fläche der Außenhülle, mit Ausnahme der Seitenversiegelungen, der Randabschnitte und des in Querrichtung mittleren Abschnitts in dem vorderen Abschnitt, dem hinteren Abschnitt und dem Schrittabschnitt der Außenhülle,

wobei die Außenschicht (12) eine äußere Oberfläche der Windel bereitstellt und die Innenschicht (13) an der Innenseite der Außenschicht angeordnet ist;

wobei die Windel außerdem ein Band für die Entsorgung aufweist, das an der äußeren Oberfläche der Außenhülle (11) bereitgestellt ist und das fest an dem in Querrichtung mittleren Abschnitt (14) angebracht ist.

2. Einweg-Höschenwindel nach Anspruch 1, wobei die Innenschicht (13) eine allgemeine Breite hat, die 1,3 bis 4,0 mal die Breite der Außenschicht (12) ist, bei getrennten und entfalteten Seitenversiegelungen und entspannter Außenschicht (12).

3. Einweg-Höschenwindel nach Anspruch 1 oder 2, wobei sowohl die Außenschicht (12) wie auch die Innenschicht (13) aus einem Vliesstoff gebildet sind, der aus thermoplastischen Fasern hergestellt ist.

4. Einweg-Höschenwindel nach einem der Ansprüche 1 bis 3, wobei die Außenschicht (12) eine Laminatschicht ist, die zumindest an der kleidungszugewandten Seite einen Durchluft-Vliesstoff aufweist.

5. Einweg-Höschenwindel nach einem der Ansprüche 1 bis 4, wobei das Verhältnis T2/T1 der Gesamtfläche T2 des verbundenen Bereichs zur Fläche T1 der der Außenhülle zugewandten Oberfläche des absorbierenden Körpers (10) mindestens 60% ist, wenn die Außenhülle (11) im entspannten Zustand ist.

6. Einweg-Höschenwindel nach einem der Ansprüche 1 bis 5, wobei der absorbierende Körper (10) und die Innenschicht (13) der Außenhülle (11) über einen Verbindungsbereich miteinander verbunden sind.

7. Einweg-Höschenwindel, die einen absorbierenden Körper (10), der einen absorbierenden Kern (4) enthält, und eine Außenhülle (11) aufweist, die mit der kleidungszugewandten Seite des absorbierenden Körpers verbunden ist, wobei die Außenhülle (11) einen vorderen Abschnitt (A) und einen hinteren Abschnitt (B) aufweist und wobei die Außenhülle (11) einen vorderen Außenhüllabschnitt (11A) und einen hinteren Außenhüllabschnitt (11B) aufweist, wobei der vordere Außenhüllabschnitt (11A) und der hintere Außenhüllabschnitt (11B) entlang ihrer beiden Seitenkantenabschnitte (A1,A2,B1,B2) miteinander verbunden sind, um ein Paar Seitenversiegelungen (S), eine Taillenöffnung (5) und ein Paar Beinöffnungen (6) zu bilden, wobei sowohl der vordere Außenhüllabschnitt (11A) wie auch der hintere Außenhüllabschnitt (11B) ein Laminat sind, das eine Außenschicht (12), die aus einer dehnbaren Schicht gebildet ist, und eine Innenschicht (13), die aus einer nicht-dehnbaren Schicht gebildet ist, aufweist, und wobei die Außenschicht (12) und die Innenschicht (13) miteinander verbunden sind in den Seitenversiegelungen (A1,A2,B1,B2), einem Randabschnitt (50) entlang der Taillenöffnung (5) und einem Randabschnitt (60) entlang jeder der Beinöffnungen (6) und entlang eines in Querrichtung mittleren Abschnitts (14) sowohl des vorderen Außenhüllabschnitts (11A) wie auch des hinteren Außenhüllabschnitts (11B) und nicht miteinander verbunden sind in der gesamten Fläche der Außenhülle (11), mit Ausnahme der Seitenversiegelungen (A1,A2,B1,B2), der Randabschnitte (50,60) und des in Querrichtung mittleren Abschnitts in dem vorderen Abschnitt und dem hinteren Abschnitt der Außenhülle, wobei die Windel außerdem ein Band für die Entsorgung aufweist, das an der äußeren Oberfläche des vorderen Außenhüllabschnitts (11A) oder des hinteren Außenhüllabschnitts (11B) bereitgestellt ist und das fest an dem in Querrichtung mittleren Abschnitt (14) angebracht ist.

8. Einweg-Höschenwindel nach Anspruch 6, wobei sowohl der vordere Außenhüllabschnitt (11A) wie auch der hintere Außenhüllabschnitt (11B) über die jeweiligen Verbindungsbereiche mit dem absorbierenden Körper (10) verbunden sind, wobei jeder der Verbindungsbereiche eine rechteckige Form hat, die sich in die Längsrichtung des absorbierenden Körpers (10) erstreckt.

9. Verfahren zur Herstellung einer Einweg-Höschenwindel, die einen absorbierenden Körper (10), der einen absorbierenden Kern (4) enthält, und eine Außenhülle (11) aufweist, die mit der kleidungszugewandten Seite des absorbierenden Körpers (10) verbunden ist, wobei die Außenhülle (11) einen vorderen Abschnitt (A) und einen hinteren Abschnitt (B) aufweist und beide Seitenkantenabschnitte (A1,A2) des vorderen Abschnitts (A) und beide Seitenkantenabschnitte (B1,B2) des hinteren Abschnitts (B) miteinander verbunden sind, um ein Paar Seitenversiegelungen (S), eine Taillenöffnung (5) und ein Paar Beinöffnungen (6) zu bilden, wobei die Außenhülle (11) ein Laminat ist, das eine Außenschicht (12), die aus einer dehnbaren Schicht gebildet ist, und eine Innenschicht (13), die aus einer nicht-dehnbaren Schicht gebildet ist, aufweist, wobei die Außenschicht (12) und die Innenschicht (13) miteinander verbunden sind in den Seitenversiegelungen (S), einem Randabschnitt (50) entlang der Taillenöffnung (5) und einem Randabschnitt (60) entlang jeder der Beinöffnungen (6) und entlang eines in Querrichtung mittleren Abschnitts (14) in dem vorderen Abschnitt (A), dem hinteren Abschnitt (B) und einem Schrittabschnitt (C) der Außenhülle (11) und nicht miteinander verbunden sind in der gesamten Fläche der Außenhülle, mit Ausnahme der Seitenversiegelungen, der Randabschnitte und des in Querrichtung mittleren Abschnitts in dem vorderen Abschnitt, dem hinteren Abschnitt und dem Schrittabschnitt der Außenhülle, und wobei die Innenschicht (13) eine allgemeine Breite hat, die größer als die Breite der Außenschicht (12) ist, bei getrennten und entfalteten Seitenversiegelungen und entspannter Außenschicht (12),
wobei das Verfahren die folgenden Schritte aufweist:

Verbinden der Außenschicht (12) in einem in Querrichtung gedehnten Zustand mit der Innenschicht (13) in einem in Querrichtung flach ausgebreiteten Zustand,
Verbinden des absorbieren Körpers (10) mit der Innenschicht (13), während die Außenschicht (12) in dem gedehnten Zustand gehalten wird,
Lösen der Außenschicht (12) von der Dehnungskraft, so dass sich die Außenschicht (12) aufgrund ihrer eigenen Zusammenziehbarkeit wieder zusammenzieht, wobei die Außenschicht (12) eine äußere Oberfläche der Windel bereitstellt und die Innenschicht (13) an der Innenseite der Außenschicht angeordnet ist; und
Bereitstellen eines Bands für die Entsorgung, das an der äußeren Oberfläche der Außenhülle oder dem hinteren Abschnitt der Außenhülle (11) bereitgestellt wird, wobei das Band fest an dem in Querrichtung mittleren Abschnitt (14) angebracht wird.

**Revendications**

1.  Couche-culotte à enfiler jetable (1) comprenant un corps absorbant (10) contenant un coeur absorbant (4) et une couverture externe (11) assemblée au côté faisant face au vêtement du corps absorbant, la couverture externe (11) comprenant une partie avant (A) et une partie arrière (B) et ayant les deux parties de bord latérales (A1, A2) de la partie avant (A) et les deux parties de bord latérales (B1, B2) de la partie arrière (B) assemblées ensemble afin de former une paire de joints latéraux (S), une ouverture de taille (5) et une paire d'ouvertures de jambe (6),
    la couverture externe (11) étant un stratifié comprenant une feuille externe (12) formée avec une feuille extensible et une feuille interne (13) formée avec une feuille non extensible,
    la feuille externe (12) et la feuille interne (13) étant assemblées entre elles dans les joints latéraux (A1, A2, B1, B2), une partie périphérique (50) le long de l'ouverture de taille (5) et une partie périphérique (60) le long de chacune des ouvertures de jambe (6) et le long d'une partie latéralement centrale (14) dans la partie avant (A), la partie arrière (B) et une partie d'entre-jambes (C) de la couverture externe (11) et non assemblées entre elles dans toute la zone de la couverture externe, excepté les joints latéraux, les parties périphériques et ladite partie latéralement centrale dans la partie avant, la partie arrière et la partie d'entre-jambes de la couverture externe,
    dans laquelle la feuille externe (12) fournit une surface extérieure de la couche, et la feuille interne (13) est disposée sur la côté interne de la feuille externe ;
    la couche comprenant en outre une bande pour le rebut prévue sur la surface extérieure de la couverture externe (11), la bande étant fermement fixée sur la partie latéralement centrale (14).

2.  Couche-culotte à enfiler jetable selon la revendication 1, dans laquelle la feuille interne (13) a une largeur représentant sensiblement 1,3 à 4,0 fois la largeur de la feuille externe (12) avec les joints latéraux retirés, la paire de joints latéraux dépliés et la feuille externe (12) détendue.

3.  Couche-culotte à enfiler jetable selon la revendication 1 ou 2, dans laquelle chacune parmi la feuille externe (12) et la feuille interne (13) est formée avec un tissu non tissé réalisé à partir de fibres thermoplastiques.

4.  Couche-culotte à enfiler jetable selon l'une quelconque des revendications 1 à 3, dans laquelle la feuille externe (12) est une feuille stratifiée ayant un tissu non tissé par passage d'air sur au moins son côté faisant face au vêtement.

5.  Couche-culotte à enfiler jetable selon l'une quelconque des revendications 1 à 4, dans laquelle la surface totale T2 de la région d'assemblage sur la surface T1 de la surface faisant face à la couverture externe du corps absorbant (10), T2/T1, représente au moins 60%, avec la couverture externe (11) dans un état détendu.

**6.** Couche-culotte à enfiler jetable selon l'une quelconque des revendications 1 à 5, dans laquelle le corps absorbant (10) et la feuille interne (13) de la couverture externe (11) étant assemblés ensemble, via une région d'assemblage.

**7.** Couche-culotte à enfiler jetable comprenant un corps absorbant (10) contenant un coeur absorbant (4) et une couverture externe (11) assemblée au côté faisant face au vêtement du corps absorbant, la couverture externe (11) comprenant une partie avant (A) et une partie arrière (B), dans laquelle la couverture externe (11) comprend une partie de couverture externe avant (11A) et une partie de couverture externe arrière (11B),
la partie de couverture externe avant (11A) et la partie de couverture externe arrière (11B) étant assemblées entre elles le long de leurs parties de bord latérales (A1, A2 ; B1, B2) pour former une paire de joints latéraux (S), une ouverture de taille (5) et une paire d'ouvertures de jambe (6),
chacune parmi la partie de couverture externe avant (11A) et la partie de couverture externe arrière (11B) étant un stratifié composé d'une feuille externe (12) formée avec une feuille extensible et une feuille interne (13) formée avec une feuille non extensible, et
la feuille externe (12) et la feuille interne (13) étant assemblées entre elles dans les joints latéraux (A1, A2 ; B1, B2), une partie périphérique (50) le long de l'ouverture de taille (5), et une partie périphérique (60) le long de chacune des ouvertures de jambe (6) et la partie latéralement centrale (14) de chacune parmi la partie de couverture externe avant (11A) et la partie de couverture externe arrière (11B), et non assemblées entre elles dans toute la zone de la couverture externe (11) excepté les joints latéraux (A1, A2 ; B1, B2), les parties périphériques (50, 60) et ladite partie latéralement centrale dans la partie avant et la partie arrière de la couverture externe,
la couche comprenant en outre une bande pour le rebut prévue sur la surface extérieure de la partie de couverture externe avant (11A) ou la partie de couverture externe arrière (11B), la bande étant fermement fixée sur la partie latéralement centrale (14).

**8.** Couche-culotte à enfiler jetable selon la revendication 6, dans laquelle chacune parmi la partie de couverture externe avant (11A) et la partie de couverture externe arrière (11B) est assemblée au corps absorbant (10) via des régions d'assemblage respectives, les régions d'assemblage ayant chacune une forme rectangulaire s'étendant dans la direction longitudinale du corps absorbant (10).

**9.** Procédé pour produire une Couche-culotte à enfiler jetable comprenant un corps absorbant (10) contenant un coeur absorbant (4) et une couverture externe (11) assemblée au côté faisant face au vêtement du corps absorbant (10), la couverture externe (11) comprenant une partie avant (A) et une partie arrière (B) et ayant les deux parties de bord latérales (A1, A2) de la partie avant (A) et les deux parties de bord latérales (B1, B2) de la partie arrière (B) assemblées ensemble afin de former une paire de joints latéraux (S), une ouverture de taille (5) et une paire d'ouvertures de jambe (6), la couverture externe (11) étant un stratifié composé d'une feuille externe (12) formée avec une feuille extensible et une feuille interne (13) formée avec une feuille non extensible, la feuille externe (12) et la feuille interne (13) étant assemblées entre elles dans les joints latéraux (S), une partie périphérique (50) le long de l'ouverture de taille (5) et une partie périphérique (60) le long de chacune des ouvertures de jambe (6) et le long d'une partie latéralement centrale (14) dans la partie avant (A), la partie arrière (B) et une partie d'entre-jambes (C) de la couverture externe (11) et non assemblées entre elles dans toute la zone de la couverture externe, excepté les joints latéraux, les parties périphériques et ladite partie latéralement centrale dans la partie avant, la partie arrière et la partie d'entre-jambes de la couverture externe, et la feuille interne (13) ayant une largeur sensiblement plus grande que la largeur de la feuille externe (12) avec les joints latéraux retirés, la paire de joints latéraux dépliés et la feuille externe (12) détendue,
le procédé comprenant les étapes consistant à :

assembler la feuille externe (12) dans un état latéralement étendu à la feuille interne (13) dans un état latéralement aplati ;
assembler le corps absorbant (10) à la feuille interne (13) tout en maintenant la feuille externe (12) à l'état étendu,
libérer la feuille externe (12) de la force d'extension pour que la feuille externe (12) se rétracte grâce à son propre pouvoir de rétraction, la feuille externe (12) fournissant une surface extérieure de la couche, et la feuille interne (13) étant disposée sur le côté interne de la feuille externe ; et
prévoir une bande pour le rebut, prévue sur la surface extérieure de la couverture externe ou la partie arrière de la couverture externe (11), la bande étant fermement fixée sur la partie latéralement centrale (14).

【Fig. 1】

【Fig. 2】

【Fig. 3】

【Fig. 4】

【Fig. 5 (a)】

【Fig. 5 (b)】

【Fig. 5 (c)】

【Fig. 6】

【Fig. 7】

【Fig. 8】

[Fig. 9]

EP 2 022 453 B1

【Fig. 10】

【Fig. 11】

【Fig. 12(a)】

【Fig. 12(b)】

【Fig. 13 (a)】

14

【Fig. 13 (b)】

14

【Fig. 14 (a)】

【Fig. 14 (b)】

【Fig. 15(a)】

15

【Fig. 15(b)】

15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3205053 A
- JP 4032718 U
- JP 62162001 A
- US 20020193774 A1
- JP 4166150 A